# EUROPÄISCHE PATENTSCHRIFT

(11) **EP 0 284 791 B1**
(45) Veröffentlichungstag und Bekanntmachung des Hinweises auf die Patenterteilung: **03.05.1995**
(21) Anmeldenummer: 88103003.5
(22) Anmeldetag: 29.02.1988
(51) Int. Cl.: C07K 14/18, A61K 39/12, C12Q 1/70, C12N 5/00

(54) **DNA- und RNA-Moleküle des westlichen Subtyps des FSME-Virus, Polypeptide, die von diesen Molekülen codiert werden, und deren Verwendung**
DNA and RNA molecules of the western-subtype TBE virus, polypeptides coded by these molecules and their use
Molécules d'ADN et d'ARN du virus de la méningoencéphalite du printemps précoce sous-type de l'ouest, polypeptides codés par celles-ci ainsi que leur utilisation

(30) Priorität: 20.03.1987 EP 87104114
(43) Veröffentlichungstag der Anmeldung: 05.10.1988
(73) Patentinhaber: IMMUNO Aktiengesellschaft, A-1221 Wien (AT)
(72) Erfinder: Heinz, Franz Xaver, Doz. Dr., A-2340 Mödling (AT); Kunz, Christian, Prof. Dr., A-1180 Wien (AT); Mandl, Christian, Mag., A-1180 Wien (AT); Dorner, Friedrich, Prof. Dr., A-1230 Wien (AT); Bodemer, Walter, Univ.-Doz. Dr., A-1228 Wien-Essling (AT)
(74) Vertreter: Grünecker, Kinkeldey, Stockmair & Schwanhäusser Anwaltssozietät

(56) Entgegenhaltungen:
- FEBS LETTERS, Band 200, Nr. 2, Mai 1986, Seiten 317-321, Federation of EuropeanBiochemical Societies; A.G. PLETNEV et al.: "Tick-borne encephalitis virusgenome. The nucleotide sequence coding for virion structural proteins"
- Idem
- EXPERIENTIA, Band 35, Nr. 5, 15. Mai 1979, Seiten 601,602; O.G. ANDZHAPARIDZEet al.: "Tick-borne encephalitis virus-specified sequences in persistentlyinfected cell culture revealed by DNA-DNA hybridization"
- VACCINE, Band 3, Nr. 3, September 1985, Seiten 292-296, Butterworth & Co.(Publishers) Ltd; J.M. LEE et al.: "Antigenic relationships between an isolateof the Western Subtype of tick-borne encephalitis virus and an inactivatedvaccine derived from it"

## Beschreibung

Die vorliegende Erfindung bezieht sich auf DNA- und RNA-Moleküle des westlichen Subtyps des FSME-Virus und Polypeptide, die durch diese Moleküle codiert werden, und deren Verwendung.

Der westliche Subtyp des Frühsommermeningoencephalitis (FSME) - Virus ist ein Mitglied der Familie der Flaviviridae, die sphärische lipidumhüllte RNA-Viren sind (Westaway et al., Intervirology 24, 183-192, 1985). Der Prototyp dieser Viren ist das Gelbfiebervirus. Definitionsgemäß sind alle Flaviviren serologisch verwandt, wie durch Hämagglutinations-Inhibitionstests gezeigt wird. Durch Kreuzneutralisation kann die Familie jedoch in mehrere Serokomplexe (DeMadrid and Porterfield, J.Gen.Virol. 23, 91-96, 1974) unterteilt werden, die Viren umfassen, die miteinander näher verwandt sind als mit Mitgliedern anderer Serokomplexe oder mit ungruppierten Flaviviren. Das FSME-Virus ist ein Mitglied des sogenannten "Tick-borne" Serokomplexes, dem außerdem das Louping-Ill, Langat, Omsker hämorrhagisches Fieber, Kyasanur Forest-Disease- und Negishi-Virus angehören.

FSME-Virusstämme konnten weiterhin einem westlichen (Europäischen) Subtyp zugeordnet werden, der häuptsächlich durch Ixodes ricinus übertragen wird, und einem fernöstlichen Subtyp mit Ixodes persulcatus als hauptsächlichem Überträger (Clarke, 1964; Bull.WHO 31, 45-56).

Diese Subtypdifferenzierung wurde sowohl durch kompetitive Radio-Immun-Assays und Peptidmapping unter Verwendung von limitierter Proteolyse der korrespondierenden Strukturglycoproteine bestätigt (Heinz und Kunz, J.Gen.Virol. 57, 263-274, 1981) als auch durch Antigenanalysen unter Verwendung von monoklonalen Antikörpern (Heinz et al., Virology 126, 525-537, 1983).

Reife Viruspartikel enthalten nur drei Strukturproteine, die als E, C und M bezeichnet werden, mit ungefähren Molekulargewichten von 50000 bis 60000, 15000 bzw. 7000.

Das Genom der Flaviviren besteht aus einzelsträngiger RNA von ungefähr 11000 Basen mit mRNA-Polarität mit einem Molekulargewicht von 4x10⁶ Dalton. Diese RNA bildet zusammen mit dem C-Protein ein sphärisches Nukleokapsid, das von einer Lipidmembran umgeben ist, die mit den Proteinen E und M assoziiert ist. Experimente unter Verwendung gereinigter Präparationen des E-Proteins, die nach Solubilisierung des Virus mit Detergentien erhalten würden, haben gezeigt, daß E das virale Hämagglutinin darstellt (d.h., es verursacht die Agglutination von bestimmten Erythrocyten unter geeigneten Bedingungen), das nach Immunisierung hämagglutinationshemmende, neutralisierende und schützende Antikörper sowie Immunität gegen Infektion mit lebendem virulenten Virus induziert (Heinz et al., Infect. Immun. 33, 250-257, 1981).

Zusätzlich zu diesen Strukturproteinen können mehrere virusspezifizierte Nichtstruktur-Proteine in Flavivirusinfizierten Zellen gefunden werden.

Die Genom-Organisation von Flaviviren ist kürzlich durch cDNA-Klonieren und Sequenzieren des Gelbfieber-, West Nil- und Murray Valley-Encephalitisvirus bestimmt worden (Rice et al., Science 229, 726-733, 1985; Dalgarno et al., J.Mol.Biol. 187, 309-323, 1986; Castle et al., Virology 147, 227-236, 1985; Castle et al., Virology 149, 10-26, 1986; Wengler et al., Virology 147, 264-274, 1985). Entsprechend diesen Analysen enthält die Genom-RNA von Flaviviren ein einzelnes langes offenes Leseraster von ungefähr 11000 Nukleotiden, das für alle Struktur-und Nichtstrukturproteine codiert.

Die Gene für die Strukturproteine sind im 5ʹ-Anteil der RNA lokalisiert; sie umfassen ungefähr ein Viertel des Genomes in der Reihenfolge C-prM(M)-E. prM stellt ein Vorläuferprotein von M dar, das in unreifen Formen von Flaviviren vorhanden ist (Shapiro et al., Virology 56, 88-94, 1973). Seine proteolytische Spaltung, die wahrscheinlich durch eine zelluläre, im endoplasmatischen Reticulum vorhandene Protease durchgeführt wird, führt zur Bildung von M, und stellt offensichtlich ein spätes Ereignis im Verlauf der Virusreifung dar.

Es wird angenommen, daß die Translation viraler Proteine am ersten oder möglicherweise zweiten AUG am 5ʹ-Ende der RNA initiiert und fortschreitet, bis sie auf ein Stopcodon am 3ʹ-Ende der RNA stößt. Man glaubt, daß die Bildung von einzelnen Proteinen durch eine Serie von spezifischen proteolytischen Spaltereignissen erfolgt, die sowohl zelluläre als auch virusspezifizierte Proteasen involvieren.

Pletnev et al. (FEBS 3660, 200, No. 2, 317-321, 1986) haben bestimmte Fragmente des fernöstlichen Subtyps des FSME-Virus kloniert und gezeigt, daß die Genom-Organisation dieses Subtyps des FSME-Virus dem der oben erwähnten Viren entspricht, aber sie konnten nicht die gesamte Sequenz der Strukturgene dieses Subtyps des FSME-Virus klonieren und sequenzieren. Darüberhinaus hat es sich gezeigt, daß die publizierte Protein E Sequenz, die vom Klon Sofjin 2 abgeleitet worden ist, nicht korrekt war, weil durch eine Verschiebung des Leserasters eine falsche Sequenz für den carboxyterminalen Bereich abgeleitet wurde.

Über 60 verschiedene Flaviviren sind bis jetzt bekannt und ungefähr zwei Drittel davon werden durch den Biß eines infizierten Arthropoden übertragen und stellen daher "Arthropode-borne" (ARBO) Viren dar. Mehrere Flaviviren sind bekannte Humanpathogene, einschließlich des Gelbfiebervirus, Denguevirus, des Japanischen Encephalitisvirus und des FSME-Virus (Shope; in: "The Togaviruses", pp. 47-82, Academic Press, New York).

Das FSME-Virus ist in vielen europäischen Ländern, in Rußland und in China endemisch. Die Krankheit ist in einigen zentraleuropäischen Ländern wie Österreich, der Tschechoslowakei oder Ungarn gut dokumentiert, und jedes Jahr werden mehrere hundert in Krankenhäuser aufgenommene Fälle registriert, die ein signifikantes Problem für die öffentliche Gesundheit darstellen.

Die Krankheit kann durch Impfen mit einem hochgereinigten formalin-inaktivierten Ganzvirus-Impfstoff (Kunz et al., J.Med.Virol. 6, 103-109, 1980), der eine Immunantwort gegen die Strukturproteine des Virus induziert, effektiv verhindert werden. Der hauptsächliche Nachteil dieses Impfstoffes ist, daß große Volumina von infektiösen und möglicherweise gefährlichen Virussuspensionen im Verlauf des Herstellungsverfahrens gehandhabt werden müssen. Daher sind weitreichende und teuere Sicherheitsvorkehrungen erforderlich.

Es war daher eine Aufgabe der vorliegenden Erfindung, die Mittel zum Herstellen eines Impfstoffes bereitzustellen, um die oben beschriebenen Nachteile zu überwinden.

In der vorliegenden Erfindung wird diese Aufgabe dadurch gelöst, daß ein DNA-Molekül, das vom westlichen Subtyp des FSME-Virus abgeleitete DNA umfaßt, die für mindestens einen Teil mindestens eines Proteins codiert, ausgewählt aus der Gruppe, die die Proteine C, prM, M oder E des westlichen Subtyps des FSME-Virus umfaßt, zur Verfügung gestellt wird.

Das beschriebene DNA-Molekül entspricht der einzelsträngigen RNA des westlichen Subtyps des FSME-Virus und ist für die Bereitstellung genetischer Information zur Expression eines Polypeptides geeignet, das die Proteine C, prM, M oder E des westlichen Subtyps des FSME-Virus einzeln oder in Kombination wie oben beschrieben sein können, oder Teile eines oder mehrerer der erwähnten Proteine, die auf den Gebieten der Diagnostik, der Therapie oder Prophylaxe sinnvoll eingesetzt werden können.

Weiterhin kann das erfindungsgemäße DNA-Molekül selbst oder Teile davon für verschiedene Zwecke verwendet werden, wie unten diskutiert werden wird.

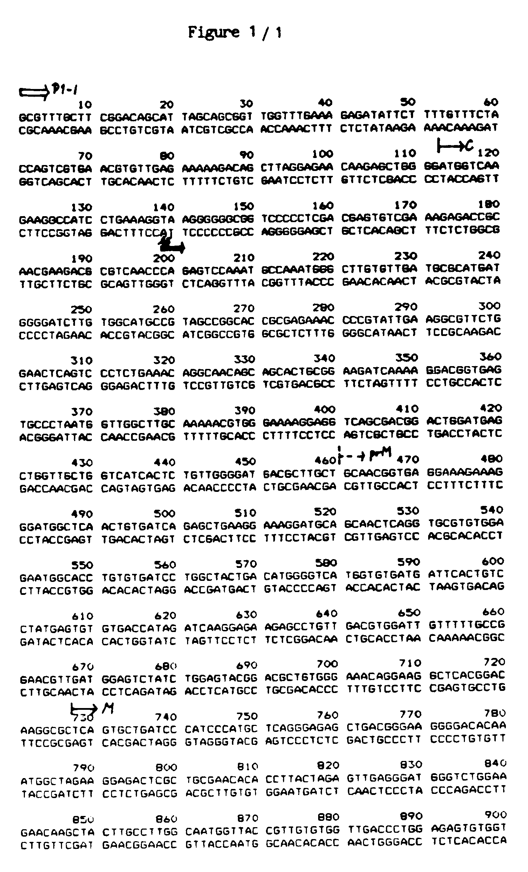

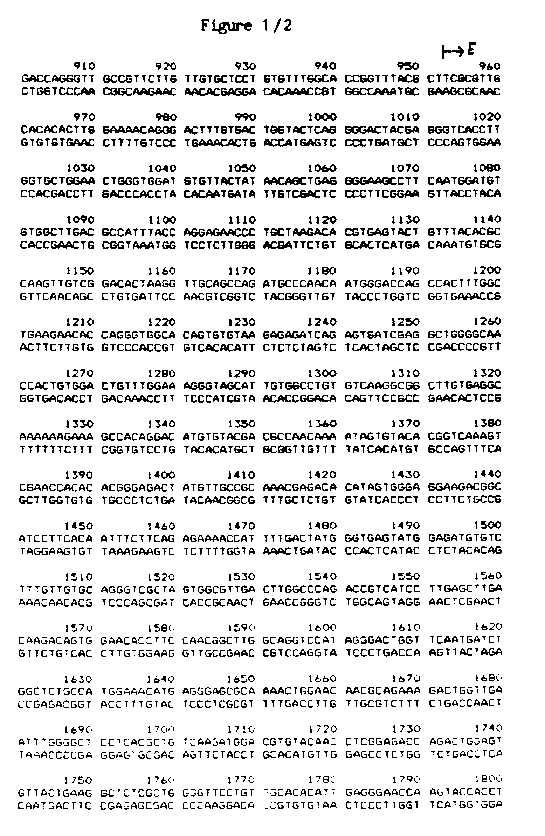

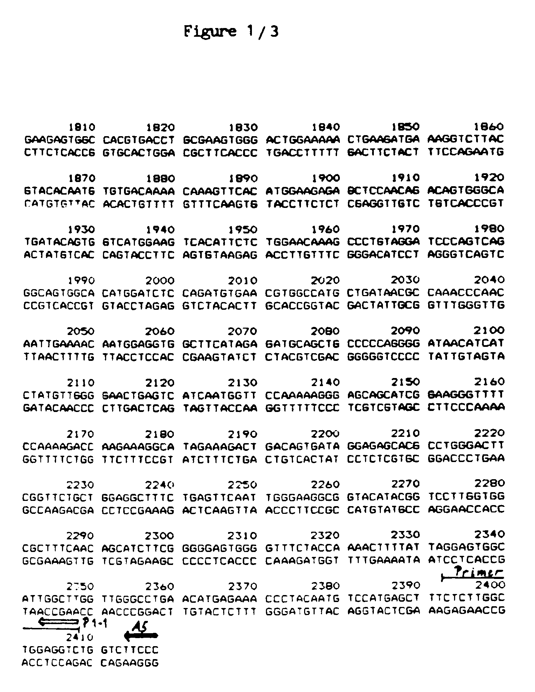

Das erfindungsgemäße DNA-Molekül kann als gesamtes Molekül verwendet werden, dessen Sequenz für die Strukturproteine des westlichen Subtyps des FSME-Virus codiert, bevorzugt so, wie in Figur 1 dargestellt. Die DNA-Sequenz, die in Figur 1 offenbart ist, zeigt das klonierte Genom des westlichen Subtyps des FSME-Virus, entsprechend dem RNA-Molekül, das im natürlich vorkommenden westlichen Subtypen des FSME-Virus enthalten ist. Die DNA-Sequenz, die in Figur 1 gezeigt ist, umfaßt den Bereich für die Strukturproteine und den 5ʹ-nichttranslatierten Bereich. Die erforschte DNA-Sequenz zeigt nur ein offenes Leseraster; die aminoterminalen Startpunkte der Proteine C, prM, M und E sowie die Grenzen der Klone A5 und P1-1 sind durch Pfeile gekennzeichnet.

Als eine bevorzugte Ausführungsform der Erfindung werden DNA-Moleküle zur Verfügung gestellt, die für ein einzelnes der Strukturproteine des westlichen Subtyps des FSME-Virus codieren, d.h., für das gesamte C, prM, M oder E Protein. Die jeweiligen Teile des beanspruchten DNA-Moleküls können direkt in geeigneten Expressionssystemen verwendet werden, um eines der gewünschten erwähnten Strukturproteine herzustellen.

Es ist eine bevorzugte Aufgabe der Erfindung, einen Teil des DNA-Moleküls, der mindestens für den Bereich einer antigenen Determinante eines der Strukturproteine des westlichen Subtyps des FSME-Virus codiert, für das Herstellen diagnostischer, therapeutisch oder prophylaktisch wirksamer Mittel zur Verfügung zu stellen. Es ist eine bekannte Tatsache, daß für die Induktion einer Antikörperantwort Bereiche mit antigenen Determinanten des Proteins verantwortlich sind. Daher müssen nicht notwendigerweise die gesamten Proteine, von denen bekannt ist, daß sie antigene Eigenschaften haben, verwendet werden, sondern es kann nur der Bereich der antigenen Determinante dieser Proteine verwendet werden. Es ist bekannt, daß in einigen Fällen eine Sequenz von nur wenigen Aminosäuren als ein Antigen wirken kann und eine Antikörperantwort verursachen kann. Daher kann es für das Herstellen eines Impfstoffes ausreichend sein, eine DNA-Sequenz zur Verfügung zu stellen, die nur für jene Aminosäuren codiert, die Funktionen einer antigenen Determinante aufweisen. Eine DNA-Sequenz, die für Bereiche der antigenen Determinanten des Strukturproteins E des westlichen Subtyps des FSME-Virus codiert, ist bevorzugt.

Es gibt zwei Wege zum Herstellen des erfindungsgemäßen DNA-Moleküls. Eine Möglichkeit, das DNA-Molekül zu erhalten, ist es, zuerst die virale RNA aus dem westlichen Subtyp des FSME-Virus zu extrahieren und das RNA-Molekül zu reinigen, gefolgt von Transkription dieser RNA-Matrize in ein DNA-Molekül unter Verwendung von Reverser Transkriptase. Eine weitere Möglichkeit ist es, die erfindungsgemäßen DNA-Moleküle chemisch zu synthetisieren, sobald die DNA-Sequenz bekannt ist.

Als eine bevorzugte Ausführungsform umfasst diese Erfindung DNA-Moleküle, die mit DNA-Molekülen entsprechend dem Hauptanspruch unter stringenten Bedingungen hybridisieren. DNA-Moleküle dieser bevorzugten Art können noch für Peptide codieren, die zum Hervorrufen einer Antikörperreaktion fähig sind, und darüberhinaus sind diese DNA-Moleküle als DNA-Sonden geeignet.

Die vorliegende Erfindung umfaßt ausdrücklich alle DNA-Sequenzen, die von den DNA-Molekülen entsprechend dem Hauptanspruch abweichen, verursacht durch Degeneration des genetischen Codes und/oder Mutationen und/oder Transpositionen, aber noch für ein Protein mit den wesentlichen antigenen Eigenschaften von mindestens einem Protein codieren, ausgewählt aus der Gruppe, die die Proteine C, prM, M oder E des westlichen Subtyps des FSME-Virus umfassen. Aus den erwähnten Gründen kann eine DNA-Sequenz, die sich von der in Figur 1 gezeigten unterscheidet, von DNA-Molekülen, die als cDNA durch Reverse Transkriptase von einer RNA-Matrize aus westlichen Subtypen des FSME-Virus hergestellt, isoliert und gereinigt werden, in hohem Maße abweichen. Nichtsdestoweniger sind die durch dieses DNA-Molekül codierten Proteinen dennoch die gleichen.

In einer bevorzugten Ausführungsform dieser Erfindung können die beanspruchten DNA-Moleküle mit zusätzlichen DNA-Sequenzen kombiniert werden.

Diese zusätzlichen Sequenzen können die Replikation und Expression des DNA-Moleküls in einer Zellkultur erlauben. Für diesen Zweck sind die wichtigsten DNA-Sequenzen diejenigen von Enhancer, Promotoren, Polyadenylierungssignalen und Splicesignalen. Diese zusätzlichen DNA-Sequenzen können mit den erfindungsgemäßen DNA-Molekülen nach Standardverfahren, die dem Fachmann vertraut sind, kombiniert werden.

Die oben für die erfindungsgemäßen DNA-Moleküle diskutierten Vorteile gelten ebenso für RNA-Moleküle. Erfindungsgemäße RNA-Moleküle können durch Isolieren und Reinigen von RNA des westlichen Subtyps des FSME-Virus oder durch rekombinante RNA/DNA-Techniken erhalten werden. Weiterhin sind nicht nur RNA-Moleküle, erhalten durch Reinigen des westlichen Subtyps des FSME-Virus, in dieser Erfindung enthalten, sondern ebenso RNA-Moleküle, die durch Transkribieren isolierter und gereinigter Virus-RNA in DNA durch Reverse Transkriptase und anschließende Transkription der so erhaltenen DNA wiederum in RNA erhalten werden.

In der vorliegenden Erfindung werden nicht nur die DNA und RNA-Moleküle wie oben beschrieben zur Verfügung gestellt, sondern außerdem Vektoren, umfassend als Insertion die diskutierten DNA- oder RNA-Moleküle, wobei der Vektor aus der Gruppe, die Plasmide, Viren und Cosmide umfaßt, ausgewählt wird. Es ist einem Fachmann bekannt, daß die beanspruchten Vektoren DNA-Sequenzen umfassen können, die die Replikation und Expression der insertierten RNA- oder DNA-Sequenzen steuern, wie z.B. Promotoren, Enhancer, etc.

Ein in der Literatur gut beschriebenes und geeignetes Plasmid für die Klonierung der genannten DNA-Sequenzen ist das Plasmid pBR322.

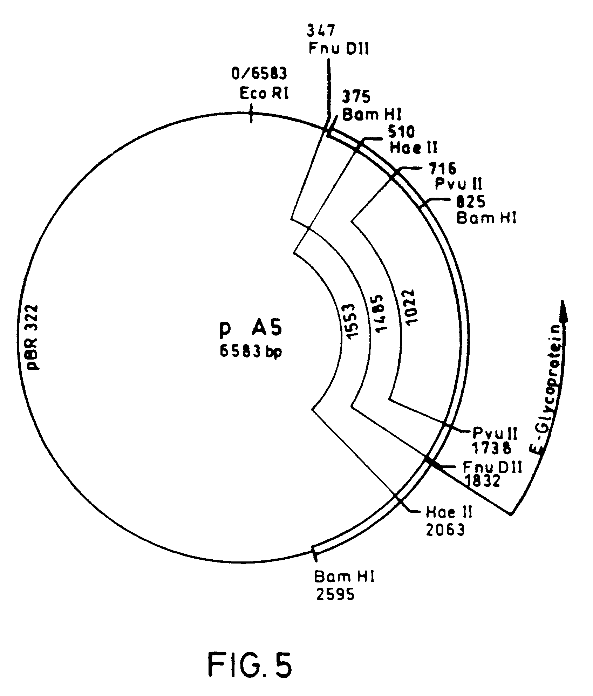

Ein bevorzugtes, auf die genannte Art hergestelltes Plasmid ist das Plasmid A5, wie es in Figur 5 dargestellt ist. Abgeleitet von dem Plasmid pBR322 enthält das Plasmid A5 die DNA-Sequenz, die für das Protein E codiert. Ein weiteres bevorzugtes Plasmid ist das Plasmid P1-1. Die genannten bevorzugten Plasmide sind bei der Deutschen Sammlung von Mikroorganismen gemäß Budapester Vertrag hinterlegt worden und haben die folgenden Hinterlegungsnummern erhalten:
a) Plasmid A5 in E. coli HB101:
   DSM 4382
b) Plasmid P1-1 in E. coli HB101:
   DSM 4383
Ausgehend von dem beschriebenen Plasmid A5 können in einer bevorzugten Ausführungsform der Erfindung Insertionsplasmide hergestellt werden, die Teile der in dem Plasmid A5 klonierten DNA-Sequenz des FSME-Virus enthalten, wobei diese Insertionsplasmide die fremde genetische Information in einer solchen Weise enthalten, daß sie durch homologe Rekombination in ein Hind III J-Fragment des Vaccinia-Virus einrekombinierbar sind. Unterfragmente des Plasmids A5 werden mit verschiedenen Restriktionsendonucleasen verdaut und in die Insertionsplasmide geklont, um beispielsweise formen des Protein E zu erhalten, die hydrophobe Aminosäuresequenzen, nämlich Signal- und Membrananker-Sequenzen entweder enthalten können oder nicht. Rekombinante Viren mit integrierter Protein E-DNA können aus Plaques isoliert werden. Die Integration der FSME-DNA-Sequenzen codierend für gewünschte Teile des Protein E wird durch Verdauen mit geeigneten Restriktionsendonucleasen und durch Hybridisierung mit ³²P-markierter FSME cRNA bestätigt und ist daran esichtlich, daß ein charakteristisches Vaccinia Hind III J Fragment nachgewiesen werden kann.

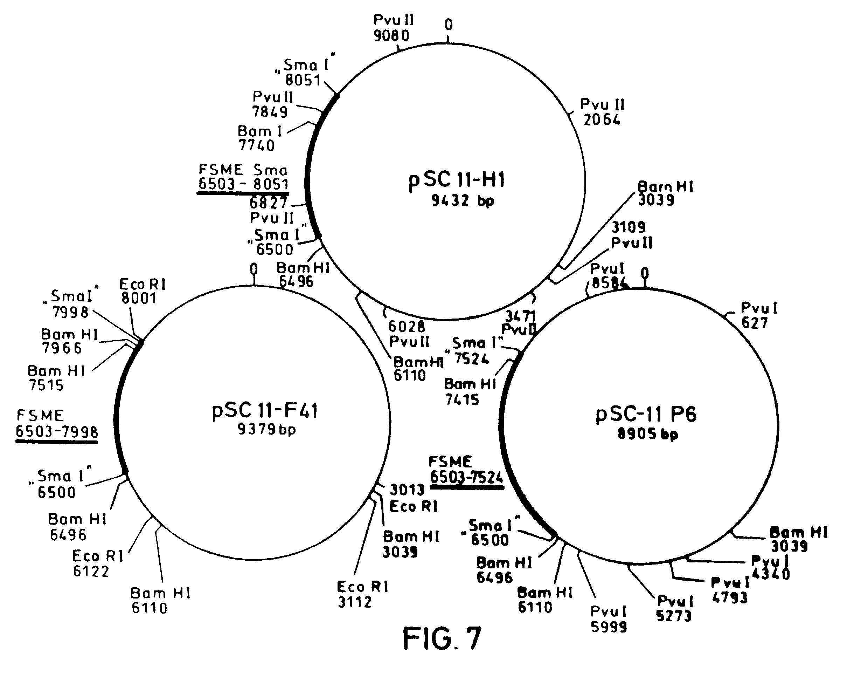

Drei Beispieie für Plasmide, die für die Durchführung der Rekombination in Vaccinia-Viren geeignet sind, sind die Plasmide pSC11-H1, das das HaeII Fragment der klonierten FSME-DNA enthält, das Plasmid pSC11-F41, das das Fnu DII-Fragment der klonierten FSME-DNA enthält, sowie das Plasmid pSC11-P6, das das PvuII-Fragment der klonierten FSME-DNA enthält. Die beschriebenen Plasmide sind in Figur 7 dargestellt. Das Ausgangsplasmid pSC11 ist bei der Deutschen Sammlung von Mikroorganismen gemäß Budapester Vertrag in E.coli unter der Hinterlegungsnummer DSM 4381 hinterlegt worden.

Die geschilderten Insertionsplasmide werden bevorzugt für das Inserieren der gewünschten Sequenzen in Vaccinia-Expressions-Vektoren verwendet.

Als Vehikel für die Sequenzen können ebenso Bakterien verwendet werden. Ein bevorzugtes Bakterium ist Salmonella typhi.

Die beanspruchten Vektoren bzw. Vehikel sind bevorzugt in Zellkulturen enthalten, in denen die Expression der Polypeptide, die durch die erfindungsgemäßen RNA- oder DNA-Sequenzen codiert werden, unter möglichst nativen Bedingungen stattfindet, wobei die Zellkultur bevorzugt eine Säugetier-Zellkultur ist. Bei der Verwendung einer Säugetier-Zellkultur werden die höchstbevorzugten Bedingungen für die Expression eines Polypeptides zur Verfügung gestellt, das als ein Impfstoff verwendet werden soll.

Besonders geeignete Zellkulturen für die Expression der gewünschten Peptide oder Polypeptide sind Vero-Zellkulturen oder Hühnerembryo-Fibroblastenzellkulturen, die einen Vaccinia-Expressions-Vektor, wie er oben beschrieben wurde, enthalten, wobei jene Proteine exprimiert werden, die den in den Vaccinia-Expressions-Vektoren enthaltenen DNA-Sequenzen entsprechen. Die rekombinanten Vaccinia-Viren können durch Southern Blot Hybridisierung auf die Anwesenheit der inserierten FSME-DNA analysiert werden.

Erfindungsgemäß werden Peptide oder Polypeptide zur Verfügung gestellt, die Aminosäuresequenzen umfassen, die durch irgendeine der oben diskutierten Nukleotidsequenzen codiert werden. Die Vorteile der Bereitstellung dieser Peptide oder Polypeptide stimmen mit den oben diskutierten Vorteilen für die DNA- und RNA-Moleküle überein.

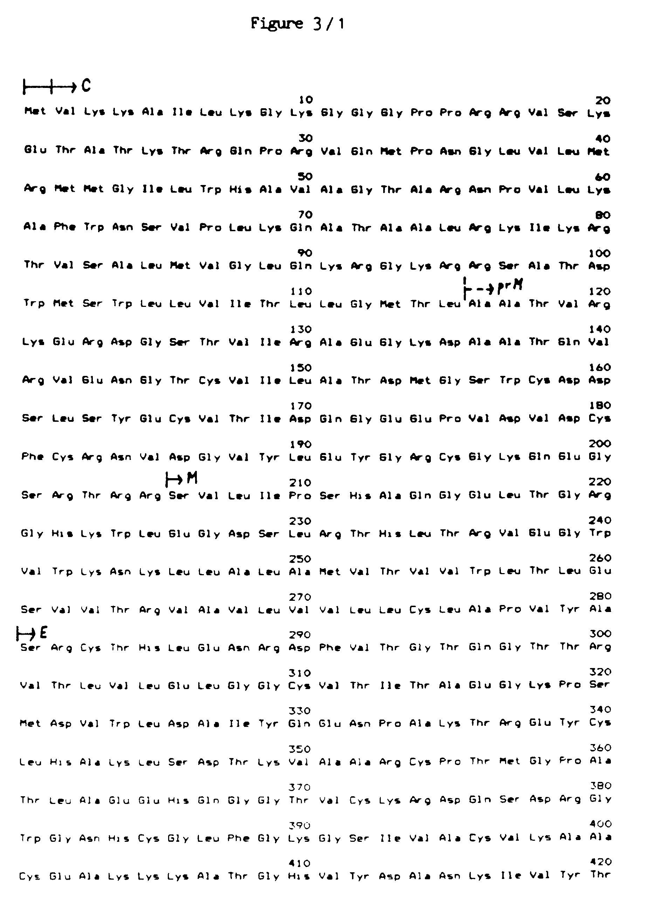

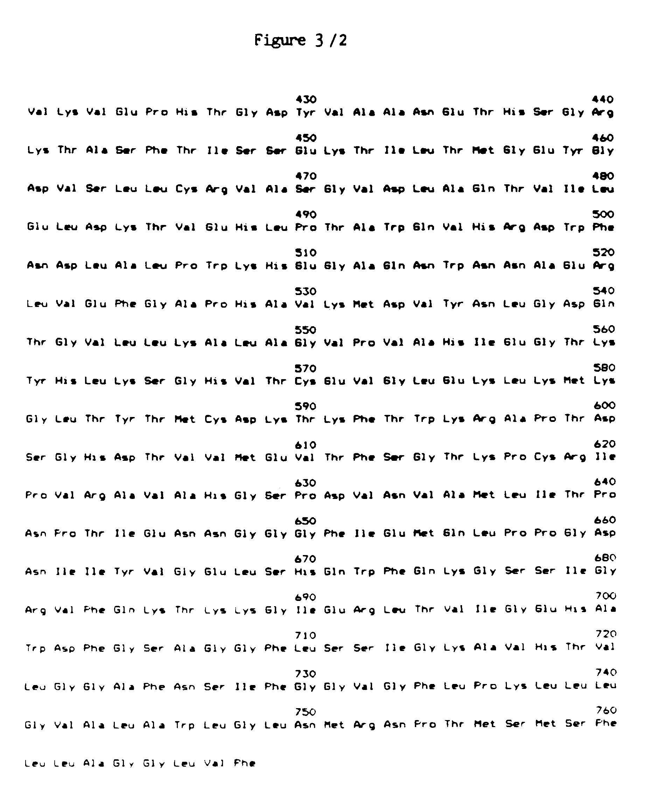

Die Aminosäuresequenzen der erfindungsgemäßen Peptide oder Polypeptide entsprechen den DNA- oder RNA-Sequenzen, die oben diskutiert worden sind, und zeigen bevorzugt die Aminosäuresequenz, die in Figur 3 dargestellt ist.

Mit den oben diskutierten Plasmiden pSC11-H1, pSC11-F41 und pSC11-P6 kann die Expression der durch sie spezifizierten Proteine bevorzugt nach dem Einrekombinieren in entsprechende Vaccinia-Expressions-Vektoren durchgeführt werden.

Obwohl es bevorzugt ist, die erfindungsgemäßen Peptide oder Polypeptide durch Expression einer der Nukleotidsequenzen dieser Erfindung herzustellen, ist es ebenso möglich, die erfinderischen Peptide oder Polypeptide chemisch zu synthetisieren.

Bevorzugt sind die erfindungsgemäßen Peptide oder Polypeptide in einer Zusammensetzung enthalten, die auf dem medizinischen Gebiet verwendet werden kann.

Die erfinderischen DNA- und RNA-Sequenzen können auch bevorzugt für die Herstellung eines Lebend-Impfstoffes verwendet werden; besonders bevorzugt werden die DNA-Sequenzen mit der DNA des Vaccinia-Virus kombiniert, das als Lebend-Impfstoff gut etabliert ist. Auf der anderen Seite kann ein Impfstoff hergestellt werden, der eine Zusammensetzung enthält, die eines oder mehrere der Peptide oder Polypeptide umfaßt, die durch die erfindungsgemäßen DNA- oder RNA-Sequenzen codiert werden.

Eine weitere bevorzugte Verwendung von Impfstoffen, die antigene Peptide oder Polypeptide enthalten, ist das Herstellen spezifischer Immunglobuline auf eine Weise, die dem Fachmann bekannt ist.

Die Zusammensetzung, die eines oder mehrere der erfindungsgemäßen Peptide oder Polypeptide umfaßt, kann ebenso als diagnostisches Mittel verwendet werden.

Die erfindungsgemäßen DNA- oder RNA-Sequenzen sind ebenso als Sonden für Identifizierungszwecke geeignet.

Die Erfindung wird durch die folgende detaillierte Beschreibung erläutert.

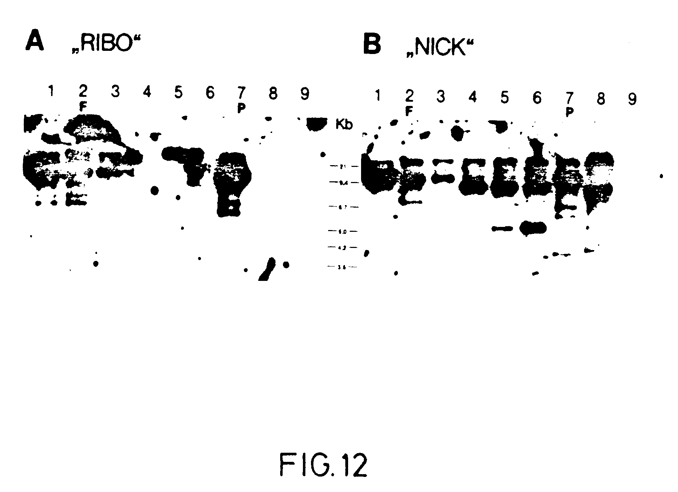

Eine bevorzugte Ausführungsform der Erfindung wird in den Figuren gezeigt, wie in den folgenden Figurenbeschreibungen dargelegt wird:
Figur 1 zeigt die DNA-Sequenz des klonierten Genoms des westlichen Subtyps des FSME-Virus, umfassend den Strukturbereich und den 5ʹ nicht-translatierten Bereich. Die aminoterminalen Startpunkte der Proteine C, prM (M), und E sowie die Grenzen der Klone A5 und P1-1 sind durch Pfeile gekennzeichnet.

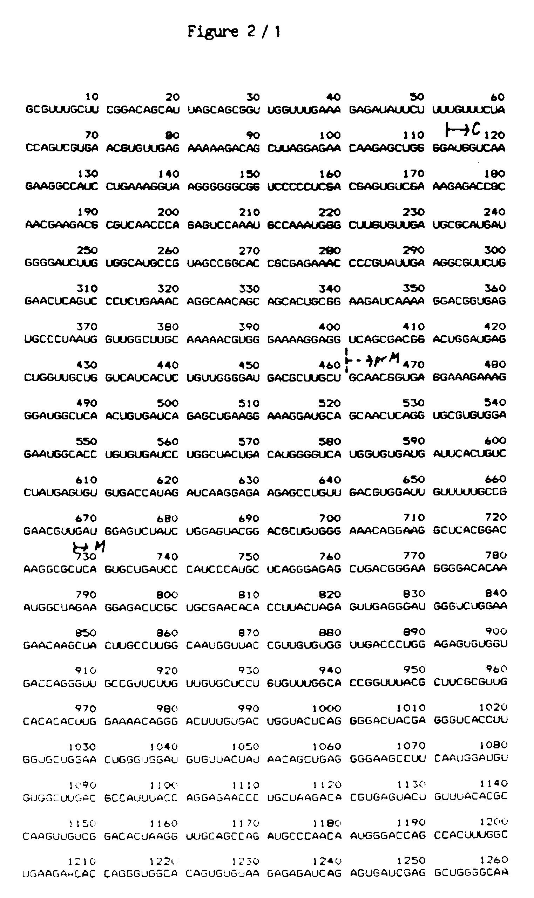

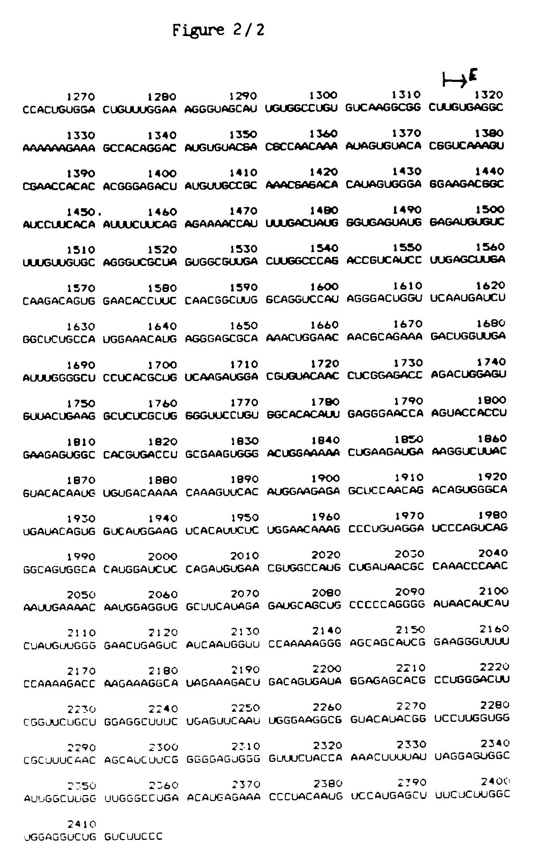

Figur 2 zeigt die RNA-Sequenz des Genomes des westlichen Subtyps des FSME-Virus, die für Strukturproteine codiert. Die aminoterminalen Startpunkte der Proteine C, prM (M) und E sind durch Pfeile gekennzeichnet.
Figur 3 zeigt die Aminosäuresequenz der Strukturproteine des westlichen Subtyps des FSME-Virus, wie sie von der DNA-Sequenz, die in Figur 1 gezeigt ist, abgeleitet wird. Die NH₂-Termini der verschiedenen Proteine sind durch Pfeile gekennzeichnet.
Figur 4 zeigt die Fotografie einer Agarosegel-Elektrophorese von Plasmiden, die für den westlichen Subtyp des FSME-Virus spezifische Inserts enthalten, nach Spaltung mit dem Restriktionsenzym SmaI (Bahnen 2-6). Die obere Bande repräsentiert die Plasmid-DNA (4363 bp), die untere Bande spezifische Insert-DNA (im Bereich von 2000 bis 3500 bp). Die DNA-Marker in den Bahnen 1 und 7 entsprechen 23130, 9416, 6557, 4361, 2322, 2027, 564 und 125 Basenpaaren (von oben nach unten).
Figur 5 zeigt ein vom Plasmid pBR322 abgeleitetes Plasmid A5, in dem Restriktionsendonuclease-Schnittstellen ausgewählter Unterfragmente dargestellt sind, wobei die entsprechenden, das FSME-Protein E codierende Regionen gekennzeichnet sind. Der Pfeil zeigt die Richtung der Transkription der genomischen RNA an.
Figur 6 zeigt das Insertionsplasmid pSC11, in dem eine einzelne SmaI Insertionsstelle, der Vaccinia-Promotor, das prokaryontische lac Z Gen und die Vaccinia TK Rekombinationssequenzen enthalten sind.
Figur 7 zeigt drei konkrete, beispielhafte FSME-Insertionsplasmide für die in vivo Rekombination in einen Vaccinia-Expressions-Vektor. Es ist die einzige SmaI-Stelle beim Nucleotid 6500 in jedem Plasmid gezeigt.
   Die jeweils erste Base der FSME-Virus spezifischen Sequenzen ist in jedem angezeigten Plasmid das Nucleotid 6503.
Figur 8 zeigt die Charakteristika der subklonierten FSME-Virus spezifischen Sequenzen. Die Translationsstart-Codons sowie hydrophobe und hydrophile Aminosäureregionen, die von einem Hydropathieplot abgeleitet sind, sind bezeichnet. Diese Regionen wurden durch ein IBI-Pustell Computerprogramm identifiziert.
   Hydrophobe Sequenzen sind mit □ , die hydrophilen Sequenzen mit ■ gekennzeichnet.
Figur 9 zeigt die physikalische Kartierung von FSME-Insertionsplasmiden sowie den Nachweis der Plasmid DNA Fragmente, die FSME spezifische Sequenzen enthalten. In A) ist das mit Ethidiumbromid gefärbte Agarose Gel mit den DNA-Fragmenten zu sehen und in B) ist das Ergebnis der Southern Blot Hybridisierung mit einer "FSME-Riboprobe"-Sonde dargestellt.
   Es ist aufgetragen in
   A)
      Spur 1 Lambda DNA geschnitten mit Hind III als Größenstandard
      Spur 2 psC11 DNA geschnitten mit PvuI
      Spur 3 pSC11-P6 DNA geschnitten mit PvuI
      Spur 4 pSC11-P6 DNA geschnitten mit BamHI
      Spur 5 pSC11 DNA geschnitten mit BamHI
      Spur 6 pSC11-F41 DNA geschnitten mit BamHI
      Spur 7 pSC11-F41 DNA geschnitten mit EcoRI
      Spur 8 pSC11 DNA geschnitten mit EcoRI
   B) Nach Hybridisierung werden FSME Sequenzen enthaltende Fragmente bei
      2,585 Kb in Spur 3
      0,919 Kb in Spur 4
      1,019 Kb in Spur 6
      1,879 Kb in Spur 7
      nachgewiesen. Diese Fragmente sind aus der Klonierung zu erwarten (siehe Fig. 7). DNA in den Spuren 1, 2, 5 und 8 hybridisiert nicht, da es sich nur um Lambda DNA oder reine pSC11 Plasmid DNA handelt.

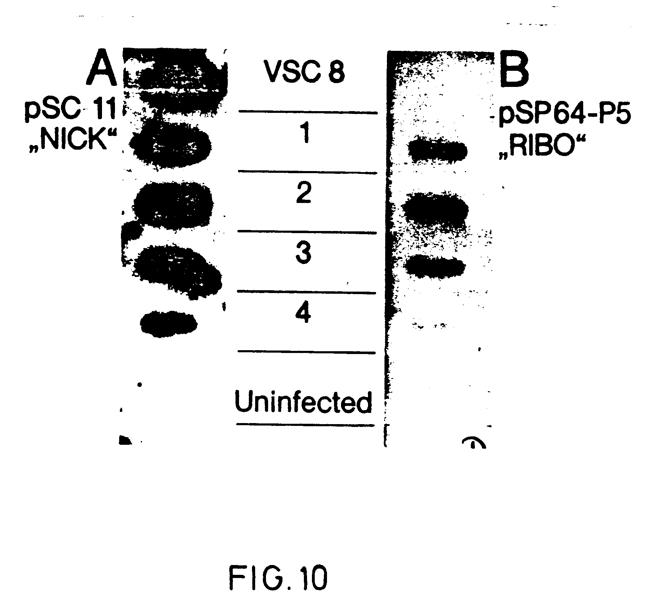

Figur 10 zeigt eine Slot Blot Hybridisierung zum Nachweis von FSME-Vaccinia Virus Rekombinanten. Nicht-infizierte Zellen, Zellen, die mit der Virus Rekombinanten vSC8 (Dr. Moss) bzw. Zellen, die mit 4 Einzelisolaten der Virus Rekombinanten P6 infiziert wurden, wurden auf Nitrozellulose Filter übertragen und mit einer ³²P-markierten pSC11 DNA (nick-repair) in A oder mit einer FSME spezifischen "Riboprobe" in B hybridisiert. Es werden keine Signale gefunden mit Zellen, die nicht infiziert wurden. Das rekombinante Virus vSC8 hybridisiert mit pSC11 wegen der TK Sequenzen. Das FSME spezifische "Riboprobe" führte bei vSC8 jedoch erwartungsgemäß zu keinem positiven Signal. Zellen, die mit Einzelisolaten der Virus Rekombinanten P6 infiziert wurden (1-4) zeigen Hybridisierung sowohl mit dem "Riboprobe" (wegen der FSME Sequenz) als auch mit der pSC11 DNA (wegen der TK Sequenz).

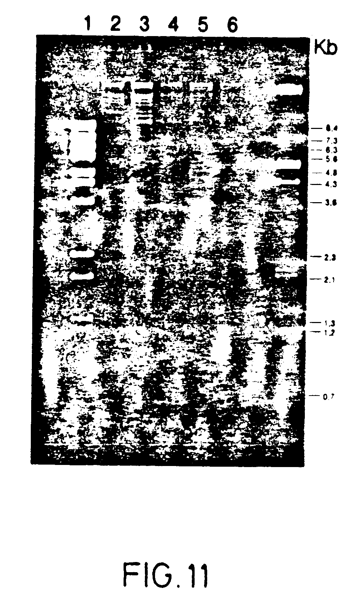

Figur 11 zeigt DNA Fragmentmuster, die nach Schneiden mit Hind III von Virion DNA aus Wildtyp Virus bzw. aus Vaccinia Virus FSME Rekombinanten erhalten wurden. Dabei wird deutlich, daß durch den Rekombinationsvorgang das Hind III J Fragment im Spaltmuster der DNA der Virus Rekombinanten verschwindet. Das ursprüngliche Hind III J Fragment mit einer Größe von ca. 5 Kb ist nur in Spur 6 des Agarosegeles zu sehen. Alle anderen DNA Präparationen enthalten das Hind III J Fragment nicht.
   Spur 1 Lambda DNA geschnitten mit BstEII als Längenstandard
   Spur 2 DNA der FSME Rekombinanten F41 (Insertionsplasmid pSC11-F41)
   Spur 3 DNA der FSME Rekombinanten P6 (Insertionsplasmid pSC11-P6)
   Spur 4 DNA einer Virus Rekombinanten P1 (Insertionsplasmid pSC11)
   Spur 5 DNA einer Virus Rekombinanten vSC8
   Spur 6 DNA aus Virionen des Wildtyp Stammes WR
Figur 12 zeigt den Nachweis von FSME spezifischen Sequenzen in Hind III DNA Fragmenten von Virus Rekombinanten durch Hybridisierung mit einem ³²P markierten FSME "Riboprobe" (A). Zur Kontrolle wurde derselbe Filter ein zweites Mal mit einer ³²P-(nick-repair) markierten pSC11 Plasmid DNA hybridisiert (B).
   A) Das FSME spezifische "Riboprobe" hybridisiert ausschließlich mit DNA aus der Virus Rekombinanten F41 in den Spuren 1, 2 und 3 und mit DNA aus der Rekombinanten P6 in Spur 7. Es ist weder mit der Rekombinanten P1 in den Spuren 4, 5 und 6 noch mit DNA aus der Rekombinanten vSC8 in Spur 8 und der DNA aus nicht infizierten Zellen in Spur 9 Hybridisierung festzustellen.
   B) Die radioaktiv markierte pSC11 DNA hybridisiert mit allen DNA Präparationen, die mit dem FSME spezifischen "Riboprobe" nachgewiesen wurden. Dies ist erklärber, da pSC11 DNA die zur Rekombination notwendigen Vaccinia TK-Sequenzen besitzt. Diese pSC11 Plasmid DNA hybridisiert aber deswegen auch mit DNA der Rekombinanten P1 in den Spuren 4, 5 und 6 sowie mit DNA der Rekombinanten vSC8 in Spur 8. DNA aus nicht infizierten Zellen hybridisiert nicht (Spur 9).

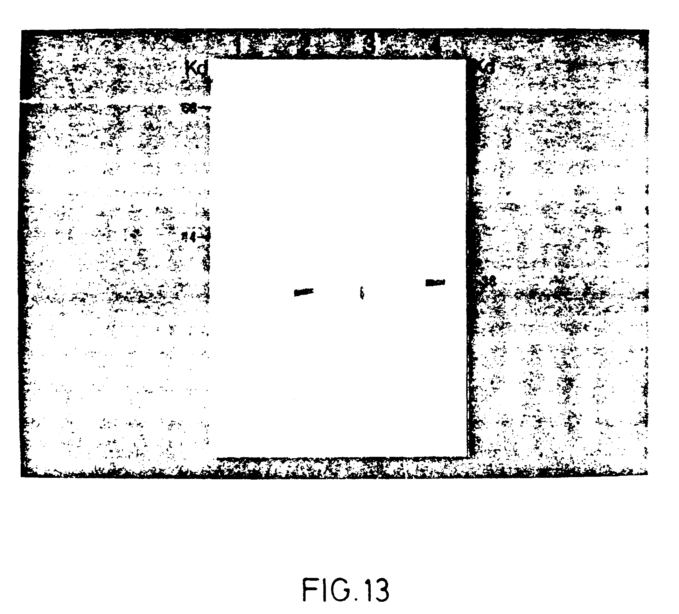

Figur 13 zeigt eine Western Blot Analyse von FSME spezifischem Protein in Extrakten aus Vero-Zellen, die mit der Virus Rekombinanten P6 infiziert wurden. Die Proteine wurden in einem 15 % Polyacrylamidgel aufgetrennt und nach Standardmethoden auf den Western Blot vorbereitet. Zur Kontrolle wurden die Proteine aus diesen infizierten Zellen mit einem negativen Humanserum inkubiert (Spur 1 und 3). Mit einem positiven Humanserum, das gegen das FSME Virus gerichtete Antikörper besitzt, konnte das zu erwartende FSME spezifische Antigen mit einer Größe von ca. 38000 D in den Spuren 2 und 4 nachgewiesen werden. Die Signale in den Spuren 1 und 3, die mit dem negativen Serum erhalten wurden, scheinen unspezifisch zu sein.

Die DNA der vorliegenden Erfindung wird hergestellt und die Sequenz wird bestimmt durch die folgenden allgemeinen Verfahren:

Zuerst wird virale RNA aus dem westlichen Subtyp des FSME-Virus oder aus mit dem westlichen Subtyp des FSME-Virus infizierten Zellen extrahiert. Unter Verwendung dieser RNA als Matrize wird eine doppelsträngige cDNA synthetisiert, zum Beispiel unter Verwendung von Reverser Transkriptase. Unter Verwendung rekombinanter DNA-Techniken wird diese cDNA in eine Vektor-DNA, so wie Escherichia coli Plasmid-DNA inseriert, um ein rekombinantes Plasmid zu erhalten. Rekombinante Plasmide werden verwendet, um geeignete Wirtszellen, wie z.B. E. coli Stamm HB101, zu transformieren, zur Amplifizierung der Plasmide oder zur Expression der entsprechenden Proteine. Einzelne Kolonien mit inserthaltigen Plasmiden werden durch die Plasmid-Mini-Präparations-Methode nach Birnboim und Doly (Nucleic Acids Research 7, 1195-1204, 1979) identifiziert. Die Basensequenz der für den westlichen Subtyp des Virus spezifischen DNA, die in dem rekombinanten Plasmid vorhanden ist, wird durch schnelle DNA-Sequenzierungsverfahren, so wie das Didesoxy-Ketten-Terminationsverfahren, bestimmt.

Eine detailliertere Beschreibung des Verfahrens des Herstellens von cDNA, rekombinanten Plasmiden, Zellen, die mit diesen Plasmiden transformiert sind, und der Bestimmung der Nukleotidsequenz ist wie folgt: zuerst kann virale RNA des westlichen Subtyps des FSME-Virus aus gereinigtem Virus erhalten werden. Zu diesem Zweck kann der westliche Subtyp des FSME-Virus in primären Hühnerembryozellen gezüchtet werden, durch Ultrazentrifugation konzentriert werden, und durch zwei Zyklen von Saccharosedichtegradienten-Zentrifugation gereinigt werden. Nach dem Solubilisieren der Proteine mit SDS in Gegenwart von Proteinase K und RNAse-Inhibitor, beispielsweise durch einstündige Inkubation bei 37° C, wird die RNA zum Beispiel mit Phenol extrahiert und mit Ethanol präzipitiert. Unter Verwendung dieser RNA als eine Matrize wird die zur Virus-RNA komplementäre DNA dann in vitro durch Reverse Transkriptase (beispielsweise aus dem "Avian Myeloblastosis" Virus) nach dem Verfahren von Gubler und Hofmann (Gene 25, 263-269, 1983) synthetisiert. Unter Verwendung von Primern, so wie Hexanukleotidprimern, erhalten aus Kalbsthymus-DNA, wird die virale RNA unter geeigneten Bedingungen, beispielsweise so, wie in dem Handbuch "cDNA Synthesis System" von Amersham (England) beschrieben, mit einer Reversen Transkriptase zusammen mit Desoxyadenosintriphosphat (dATP), Desoxythymidintriphosphat (dTTP), Desoxyguanosintriphosphat (dGTP) und Desoxycytidintriphosphat (dCTP) als Substraten inkubiert. Das so erhaltene cDNA.RNA-Hybrid wird dann mit RNAse H unter definierten Bedingungen behandelt, unter denen in der RNA des Hybridmoleküls Nicks erzeugt werden. Anschließend kann die E. coli DNA Polymerase I verwendet werden, um den RNA-Strang effizient zu ersetzen, wobei die genickte RNA als Primer dient. Die doppelsträngige so erhaltene DNA wird durch Phenol-Chloroform-Extraktion deproteinisiert, mit Ethanol präzipitiert und verbleibende RNA-Fragmente werden durch Behandlung mit RNAse (z. B. in TE-Puffer, 30 min bei 37°C) entfernt.

Das Klonieren der dsDNA wird beispielsweise unter Verwendung von synthetischen Adaptoren durchgeführt. Für diesen Zweck wird die dsDNA mit dem Klenow-Fragment der E. coli DNA-Polymerase I unter geeigneten Bedingungen (Maniatis et al., Molecular Cloning, CSH 1982) behandelt, um eine maximale Menge von klonierbaren glatten Enden sicherzustellen, gefolgt von Phenolextraktionen für die Deproteinisierung. Die dsDNA mit den glatten Enden wird am 5ʹ Ende mit Polynucleotidkinase nach Standardverfahren (Maniatis et al., Molecular Cloning, 1982) phosphoryliert. Die phosphorylierte dsDNA wird unter geeigneten Bedingungen mit BamHI-SmaI-Adaptoren in Gegenwart von DNA-Ligase inkubiert. Die Reaktionsmischung wird auf ein 1 %-iges Agarosegel geladen, aufgetrennt und verschiedene Größenbereiche von mit Adaptoren verbundener cDNA werden aus dem Gel ausgeschnitten und gereinigt, beispielsweise durch Elektroelution. Die so gewonnene DNA wird nochmals mit Polynucleotidkinase wie zuvor beschrieben phosphoryliert. Auf der anderen Seite wird die als Vektor-DNA zu verwendende Plasmid-DNA, beispielsweise E. coli Plasmid pBR322-DNA, mit der Restriktionsendonuclease BamHI gespalten und unter Verwendung von bakterieller alkalischer Phosphatase dephosphoryliert. Die synthetische dsDNA, die BamHI-Adaptoren an beiden Enden enthält, und die BamHI-geschnittene Vektor-DNA werden unter geeigneten Bedingungen inkubiert, um eine Hybridisierung komplementärer überhängender Sequenzen an den Enden der beiden DNA-Moleküle zu erlauben, und unter Verwendung von T4-DNA Ligase ligiert. Das rekombinante DNA-Molekül wird dann verwendet, um einen kompetenten E. coli Stamm (beispielsweise E. coli HB101) wie von Maniatis et al. (Molecular Cloning CSH, 1982) beschrieben, zu transformieren. Die verwendete Vektor-DNA enthält zwei Gene, die Resistenz gegen Ampicillin bzw. Tetracyclin vermitteln. Bei dem verwendeten Klonierungsverfahren wird das Tetracyclin-Resistenz-Gen zerstört. Für die Selektion von Rekombinanten, welche wahrscheinlich virusspezifische Inserts enthalten, werden die transformierten Bakterien in Gegenwart von Ampicillin gezüchtet und dann auf Tetracyclinsensitivität analysiert. Plasmide tetracyclinsensitiver Kolonien werden mit dem Plasmid-Mini-Präparationsverfahren (Birnboim und Doly, Nucleic Acids Research 7, 1195-1204, 1979) isoliert und die Insertgrößen werden mit Restriktions-Enzym-Analysen unter Verwendung von SmaI und Trennung der erhaltenen Fragmente auf 1 %-igen Agarosegelen analysiert.

Die Basensequenz dieser Inserts wird mit der Didesoxy-Ketten-Terminationsmethode nach Sanger et al. (Proc.Natl.Acad.Sci. U.S.A. 74, 5463-5467, 1977) bestimmt. Die erhaltene Sequenz wird auf Homologien mit bereits bekannten Sequenzen anderer Flaviviren (Rice et al., Science 229, 726-733, 1985; Dalgarno et al., J.Mol.Biol. 187, 309-323, 1986; Castle et al., Virology 147, 227-236, 1985; Castle et al., Virology 149, 10-26, 1986; Wengler et al., Virology 147, 264-274, 1985; Pletnev et al., FEBS 3660 200, No. 2,317-321, 1986) mit Hilfe von Computerprogrammen untersucht, um die untersuchte Sequenz in der Gesamtsequenz der genomischen RNA zu lokalisieren. Figur 1 zeigt die vollständige Nukleotidsequenz und Figur 3 die korrespondierende Aminosäuresequenz der strukturellen Region des westlichen Subtyps des FSME-Virus-Genoms.

Die genauen Aminotermini von E und M werden durch N-terminale Aminosäuresequenzanalysen bestimmt, beispielsweise unter Verwendung des manuellen Verfahrens, das durch Chang et al. (FEBS Letters 93, 205-214, 1978) beschrieben wird. Für diesen Zweck wird das E-Protein isoliert, beispielsweise durch präparative SDS-PAGE, aus dem Gel elektroeluiert und einer N-terminalen Sequenzanalyse unterworfen. Die resultierende Sequenz ist Ser-Arg-Cys, daher liegt der Amino-Terminus von E an exakt der gleichen Position wie der von anderen Flaviviren, d.h., zwei Aminosäuren vor dem ersten und hochkonservierten Cysteinrest (Fig. 3).

Der folgende Weg kann eingeschlagen werden, um den Aminoterminus des M-Proteins zu bestimmen. Gereinigtes Virus wird mit einem nicht-ionischen Detergenz (z.B. Triton X 100) solubilisiert und Proteinkomplexe, die E und M enthalten, werden durch Dichtegradientenzentrifugation in einem detergenzfreien Saccharosegradienten (Heinz und Kunz, J.Gen.Virol. 49, 125-132, 1980) gewonnen. Die Sequenzanalyse dieser Proteinkomplexe führt zu der folgenden Doppelsequenz:
Ser-Arg-Cys-
Ser-Val-Leu-
wobei Ser-Arg-Cys der Sequenz von E entspricht und Ser-Val-Leu das aminoterminale Ende des M-Proteins darstellt. Diese Termini sind in den in den Figuren 1 und 3 gezeigten Sequenzen gekennzeichnet.

Die vorliegende Erfindung stellt daher das erste Mal die Nukleotidsequenz des 5ʹ Bereiches des westlichen Subtyps des FSME-Virus-Genoms zur Verfügung, einschließlich der Sequenzen, die für Strukturproteine codieren, und, als Folge davon, stellt sie außerdem die Aminosäuresequenzen dieser Strukturproteine zur Verfügung.

Die Basensequenz aus Figur 1 ist ein bevorzugtes Beispiel für eine DNA, die für Polypeptide mit den Eigenschaften von Strukturproteinen des westlichen Subtyps des FSME-Virus codiert. Infolge der Degeneration des genetischen Codes kann die gleiche Aminosäuresequenz ebenso durch andere als die in der Figur gezeigten Basentriplets codiert werden.

Im Schutzbereich dieser Erfindung liegen ebenso Sequenzen, die durch Mutation, Transposition und Degradation verändert sind, die nichtsdestoweniger für eine Aminosäuresequenz codieren, die noch die wesentlichen antigenen Charakteristika der Strukturproteine aufweisen.

Außerdem bezieht sich diese Erfindung nicht nur auf die exakt gleiche Aminosäuresequenz wie diejenige, die in Figur 3 gezeigt ist, die nur eine beispielhafte Sequenz eines natürlichen Isolates des westlichen Subtyps des FSME-Virus darstellt. Es ist eine gut bekannte Tatsache, daß die Genome von RNA-Viren höheren Mutationsfrequenzen unterliegen, als jene, die für DNA-Viren oder zelluläre Gene gefunden werden, infolge der hohen Fehlerrate der RNA-Polymerasen und des Fehlens eines Korrekturmechanismus (Holland et al., Science 215, 1577-1585, 1982; Reanney, Ann. Rev. Microbiol. 36, 47-73, 1982). In Abhängigkeit von den Charakteristika des Virus können diese Mutationen zu der schnellen Entwicklung eines neuen Virustypes infolge von Antigen-Drift führen, wie das beim Influenza-Virus der Fall ist (Both et al. in: "The Origin of Pandemic Influenza Viruses", W.G. Laver (ed.), Elsevier, 1983). Auf der anderen Seite können RNA-Viren in Bezug auf ihre Antigenstruktur unter natürlichen ökologischen Bedingungen stabiler sein, vermutlich als Folge funktioneller Zwänge, die keine weitreichenden strukturellen Veränderungen in antigenaktiven Proteinen zulassen. Nichtsdestoweniger muß ein gewisses Maß an Variation berücksichtigt werden und dies kann durch Sequenzvergleiche verschiedener natürlicher Isolate gezeigt werden.

Dies kann zum Beispiel durch Sequenzieren der RNA verschiedener Isolate unter Verwendung der Didesoxy-Ketten-Terminationsmethode mit synthetischen Oligonukleotiden als Primern durchgeführt werden, die gemäß der Sequenz des Prototypisolates, das in Figur 1 gezeigt ist, hergestellt worden sind.

Die Analyse der mit dem E-Protein korrespondierenden Nukleotidsequenzen solcher verschiedenen Isolate hat unterschiedliche Nukleotide aufgezeigt. Die folgenden Nukleotidaustausche wurden in der RNA eines natürlichen Isolates (ZZ-9) im Vergleich mit Neudörfl gefunden:

| Position des Nukleotides | FSME | ZZ-9 | Aminosäureaustausch |
|---|---|---|---|
| 2375 | A | U | Thr -- Ser |
| 2347 | U | C | -- |
| 2323 | A | G | -- |
| 2317 | A | G | -- |
| 2281 | C | U | -- |
| 2266 | U | C | -- |
| 2263 | A | G | -- |
| 2021 | C | U | -- |
| 1876 | C | U | -- |
| 1868 | A | G | Met -- Val |
| 1773 | C | U | Ala -- Val |
| 1668 | A | G | Glu -- Gly |
| 1663 | C | U | -- |
| 1661 | A | G | Asn -- Asp |
| 1660 | C | U | -- |
| 1472 | U | C | -- |
| 1451 | A | G | Ile -- Val |
| 1348 | C | U | -- |
| 1114 | U | C | -- |
| 1111 | U | C | -- |
| 1090 | C | U | -- |
| 1048 | U | C | -- |
| 994 | U | C | -- |

Die beobachteten Austausche sind ein Ausdruck der natürlichen Vatiationsrate des FSME-Virus, die nicht zu einem neuen Serotyp führt. Sequenzhomologien zwischen den E-Proteinen von Flaviviren, die nicht zu demselben Serokomplex gehören, liegen im Bereich von 40 bis 50 %, beispielsweise 44,4 % Homologie zwischen YF und MVE Virus, und im Bereich von 70 bis 80 % zwischen Mitgliedern des gleichen Flavivirus-Serokomplexes, z.B. 77,1 % Homologie zwischen WN und MVE Virus.

Ein Vergleich der publizierten Nukleotidsequenzen des fernöstlichen Subtyps (Pletnev et al., FEBS 3660 200, No.2, 317-321, 1986) und des westlichen Subtyps wies eine Homologie von ungefähr 85 % auf.

Alle Sequenzen, die im Vergleich zu den Prototypsequenzen leichte Variationen zeigen, so wie sie in anderen natürlichen Isolaten des westlichen Subtyps des FSME-Virus auftreten, sind in dieser Erfindung enthalten. Solche Variationen können außerdem durch in vitro-Modifikation der Nukleinsäure erhalten werden. Die Erfindung umfaßt daher alle Sequenzen, die unter stringenten Bedingungen hybridisieren, beispielsweise bei mindestens 90 % Nukleotidsequenzhomologie mit den offenbarten Sequenzen oder Teilen von Sequenzen, die bevorzugt für Proteine oder Peptide mit den wesentlichen Charakteristika der antigenen Determinanten der Strukturproteine des westlichen Subtyps des FSME-Virus codieren.

Es ist dem Fachmann gut bekannt, die offenbarte DNA oder Teile davon in geeignete Vektoren zu insertieren und die rekombinanten Vektoren für die Transformation geeigneter Zellen zu verwenden, entweder für die Amplifikation der DNA oder um Expression der korrespondierenden Proteine zu erhalten. Geeignete Wirte, Vektoren und Bedingungen für diese Operationen sind dem Fachmann gut bekannt. Es gibt viele Publikationen, die die Synthese fremder Proteine mittels rekombinanter DNA-Technologie beschreiben (einen Überblick gibt beispielsweise "Maximizing Gene Expression" (Reznikoff and Gold, eds.), Butterworths, 1986; Harris, Gen. Eng. 4, 127-183 (Williamson, Ed.) Academic Press, 1983; Wetzel and Goeddel, The Peptides 5, 1-64, 1983). Mittels dieser Verfahren können von klonierter DNA codierte Proteine entweder in Bakterien, in Hefe oder in Säugetierzellen exprimiert werden. Es ist weiterhin Stand der Technik, solche exprimierten Proteine als Impfstoffe für Immunisierungszwecke (Valenzuela et al., Nature 298, 347-350, 1982; McAleer et al., Nature 307, 178-180, 1984) oder als diagnostische Antigene zu verwenden.

Fremde DNA- oder RNA-Sequenzen können ebenso in die Genome lebender Viren eingeführt werden, wodurch rekombinante Viren erzeugt werden, die als Lebend-Impfstoff verwendet werden können (Review von Mackett and Smith, J.Gen.Virol. 67, 2067-2082, 1986).

Ebenso kann eine Kombination verschiedener Gene, beispielsweise abgeleitet von verschiedenen Viren, gleichzeitig durch rekombinante DNA-Technologie exprimiert werden und für die Impfung verwendet werden (Perkus et al., Science 229, 981-984, 1985). Die Erfindung schließt daher alle Kombinationen von Sequenzen der offenbarten Sequenzen mit anderen Sequenzen ein, wie z.B. Gene, die für andere Proteine codieren, oder Sequenzen, die zur Expression der Proteine beitragen, wie z.B. Promotoren, Enhancer, Polyadenylierungs- oder Splicesignale.

Es ist dem Fachmann gut bekannt, daß nicht notwendigerweise die gesamten natürlich auftretenden Proteine für die Immunsisierung oder diagnostische Zwecke verwendet werden müssen ( Lerner, Nature 299, 592-596, 1982; Arnon, TIBS 11, 521-524, 1986). Im Fall des westlichen Subtyps des FSME-Virus E-Proteins wurde beispielsweise gezeigt, daß hämagglutinationshemmende und neutralisierende Antikörper durch Immunisierung mit einem proteolytischen Fragment von 9000 Dalton induziert werden können, das ebenso mit polyklonalen Immunseren reagiert, die durch Immunisieren mit dem gesamten natürlichen Protein erhalten worden sind (Heinz et al., J.Gen.Virol. 65, 1921-1929, 1984). Andere proteolytische Fragmente können ebenso für die Immunisierung oder diagnostische Zwecke geeignet sein.

Proteine oder Teile von Proteinen, die als Impfstoffe oder diagnostische Mittel verwendet werden, können nicht nur durch rekombinante DNA-Technologien wie oben beschrieben hergestellt werden, sondern die in der vorliegenden Erfindung offenbarte Sequenzinformation kann ebenso Grundlage für die chemische Synthese von Oligopeptiden sein. Auf diesem Gebiet gibt es eine umfassende Literatur: synthetisierte Peptide sind entsprechend DNA-Sequenzen hergestellt worden, die für viele verschiedene Proteine codieren, und diese sind für eine Vielzahl von Zwecken wie z.B. molekularbiologische und immunologische Studien (Lerner et al., Cell 23, 309-310, 1981; Lerner, Nature 299, 592-596, 1982) oder Impfungen (Shinnick et al., Ann.Rev.Microbiol. 37, 425-446, 1983; DiMarchi et al., Science 232, 639-641, 1986) verwendet worden. Die Herstellung und Verwendung von Peptiden oder Kombinationen von Peptiden, die den in der vorliegenden Erfindung offenbarten Sequenzen entsprechen, stellen daher den Stand der Technik dar.

Es ist dem Fachmann weiterhin bekannt, die Nukleinsäuren und Sequenzen, die durch die vorliegende Erfindung zur Verfügung gestellt werden, für die Herstellung von Hybridisierungssonden, beispielsweise zum Bestimmen von Virus-RNA in Zecken oder Körperflüssigkeiten, zu verwenden (Meinkoth und Wahl, Anal.Biochem. 138, 267-284, 1984; Kulski und Norval, Arch.Virol. 83, 3-15, 1985). Diese können entweder durch rekombinante DNA-Technologien oder durch chemische Synthese von Oligonukleotiden entsprechend den offenbarten Sequenzen hergestellt werden.

Die folgenden Beispiele erläutern die Erfindung:

### Beispiel 1:

### Propagieren und Reinigen des FSME-Virus

Eine 10 %-ige Suspension von mit dem westlichen Subtyp des FSME-Virus infizierten Saugmausgehirnen wurde für die Infektion von primären Hühnerembryozell-Monolayerkulturen, die in mit 15 mM HEPES und 15 mM EPPS bei pH 7,6 gepufferten Minimalmedium (MEM) gehalten wurden, verwendet. Nach 40 h Inkubation bei 37°C wurde der Überstand 30 min bei 4°C und 10000 g geklärt und das Virus durch 3 h Ultrazentrifugation bei 4°C und 50000 g pelletiert. Das Virus wurde dann in einem geeigneten Volumen von TAN-Puffer (0,05 M Triethanolamin, 0,1 M NaCl, pH 8,0) resuspendiert und 110 min einer Zonal-(rate-zonal) Zentrifugation in einem 5 - 20 %-igen (w/w) Saccharosedichtegradienten bei 170000 g bei 4°C unterworfen. Der Viruspeak wurde durch Scannen des Gradienten bei 254 nm lokalisiert und einer Gleichgewichts-Dichtegradienten-Zentrifugation in einem 20 - 50 %-igen (w/w) Saccharosegradienten 18 h bei 4°C und 150000 g unterworfen. Der Viruspeak wurde gegen TAN-Puffer pH 8,0 dialysiert, um überschüssige Saccharose zu entfernen.

### Beispiel 2:

### Herstellen viraler RNA

100 ug gereinigtes Virus wurden in 400 ul eines Proteinase K Reaktionspuffers (10 mM Tris pH 7,8, 5 mM EDTA, 0,5 % w/v SDS) verdünnt. Proteinase K wurde bis zu einer Endkonzentration von 200 ug/ml zugefügt und die Mischung wurde 1 h bei 37°C inkubiert. Anschließend wurde die Lösung deproteinisiert durch zweifache Extraktion mit dem gleichen Volumen (400 ul) Phenol und einfache Extraktion mit Chloroform: Isoamylalkohol (24:1). Dann wurden 26 ul einer 3 M Natriumacetatlösung zugefügt und die RNA wurde mit 2,5 Volumen Ethanol präzipitiert. Vor der weiteren Verwendung wurde ein Aliquot der RNA mit Glyoxal denaturiert und auf einem 1 %-igen Agarosegel aufgetrennt, um die Ausbeute und die Qualität der Präparation zu prüfen.

### Beispiel 3

### Synthese doppelsträngiger cDNA

5 ug ethanolpräzipitierter RNA wurden in 40 ug eines Erststrang-Synthesepuffers (Amersham, UK), der 5 ug eines "Random" Oligonukleotidprimers enthielt, resuspendiert, eine Minute auf 70°C erhitzt und dann langsam auf Raumtemperatur abkühlen gelassen. Alle vier Desoxynukleotid-Triphosphate wurden bis zu einer Endkonzentration von 1 mM zugefügt. Weiterhin wurden 5 Einheiten humanen Plazenta-Ribonukleaseinhibitors und 10 uCi α-³²P dCTP der Mischung zugesetzt. Die Erststrang-Synthese wurde durch die Addition von 100 Einheiten Reverser Transkriptase (Amersham) gestartet und für eine Dauer von 2 h bei 42°C durchgeführt. Dann wurde die Reaktionsmischung auf Eis gestellt und die Reagenzien für die Zweitstrangsynthese wurden in der folgenden Reihenfolge zugefügt: 93,5 ul eines Zweitstrangsynthesepuffers (Amersham, UK), 4 Einheiten Ribonuklease H, 23 Einheiten E. coli DNA Polymerase und Wasser bis zu einem Endvolumen von 250 ul. Die Zweitstrangsynthese wurde durch aufeinanderfolgende Inkubationen bei 12°C und 22°C (jeweils 2 h) durchgeführt und durch 20-minütiges Erhitzen der Lösung auf 70°C abgestoppt. Die doppelsträngige cDNA wurde mit 10 Einheiten T4 DNA Polymerase 30 min bei 37°C inkubiert, um glatte Enden zu erzeugen. Schließlich wurde die cDNA durch Phenol- und Chloroformextraktionen gereinigt und mit 2 Volumen Ethanol präzipitiert.

### Beispiel 4

### Adaptorenligation

Die cDNA wurde mit Polynukleotidkinase in Gegenwart von zwei mM ATP (Maniatis et al., Molecular Cloning, CSH 1982) behandelt, um sicherzustellen, daß alle 5ʹ Enden phosphoryliert waren und daher ligiert werden konnten. BamHI-SmaI Adaptoren wurden von Pharmacia bezogen. Diese Adaptoren wiesen ein 5ʹ-überstehendes, mit BamHI kompatibles überstehendes (sticky) Ende und ein glattes Ende auf, und sie enthielten die vollständige SmaI-Erkennungsstelle innerhalb ihrer Sequenz. Zuerst wies nur das glatte Ende eine 5ʹ-Phosphatgruppe auf und konnte ligiert werden, während das überstehende Ende dephosphoryliert war. 5 ug dieser Adaptoren wurden mit der cDNA in 20 ul des Ligationspuffers (50 mM Tris, pH 7,5, 5 mM MgCl₂, 5 mM DTT, 1 mM ATP) gemischt. Die Reaktion wurde durch T4 DNA Ligase (New England Biolabs) in einer Übernachtreaktion bei 14°C katalysiert.

### Beispiel 5:

### Größenauftrennung der cDNA

Die cDNA wurde auf einem 1 %-igen niedrigschmelzenden Agarosegel aufgetrennt. Verschiedene Größenklassen wurden aus dem Gel ausgeschnitten und die DNA nach Standardverfahren aus der Agarose mit heißem Phenol extrahiert. Trotz der geringen Menge der gehandhabten DNA, die durch Anfärben mit Ethidiumbromid kaum detektiert werden konnte, konnten die Extraktionsschritte mit Hilfe der in die cDNA inkorporierten radioaktiven Markierung leicht verfolgt werden. Der Größenauftrennungsschritt ermöglichte das selektive Klonieren großer cDNA-Fragmente und diente außerdem dazu, die cDNA vollständig von unligierten Adaptormolekülen zu trennen.

### Beispiel 6:

### Klonieren in ein bakterielles Plasmid

Die BamHI-kompatiblen Enden der cDNA verschiedener Größenklassen wurden mit Polynukleotidkinase phosphoryliert und die Deproteinierung wurde durch Phenol- und Chloroformextraktionen durchgeführt. Dann wurde die DNA mit 2 Volumen Ethanol präzipitiert und in einem kleinen Volumen Wasser resuspendiert. 20 - 30 ng der cDNA wurden mit 100 ng des Escherichia coli Plasmids pBR322, das durch BamHI-Spaltung linearisiert und dephosphoryliert worden war, um Selbstligation zu verhindern, gemischt. Diese beiden Komponenten wurden mit T4 DNA Ligase in 5 ul des Ligationspuffers (siehe oben) in einer Übernachtreaktion bei 16°C ligiert. Am nächsten Tag wurde die Ligationsmischung mit Wasser fünffach verdünnt und dann direkt verwendet, um E. coli HB101 Zellen zu transformieren. Kompetente Bakterien wurden von BRL (Maryland, USA) bezogen und genau entsprechend dem vom Hersteller zur Verfügung gestellten Protokoll mit 5 ul der verdünnten Ligationsmischung transformiert. Typischerweise wurden nach Plattieren auf ampicillinhaltige Agarplatten mehrere hundert transformierte Zellen erhalten.

### Beispiel 7:

### Identifizierung inserthaltiger Plasmide

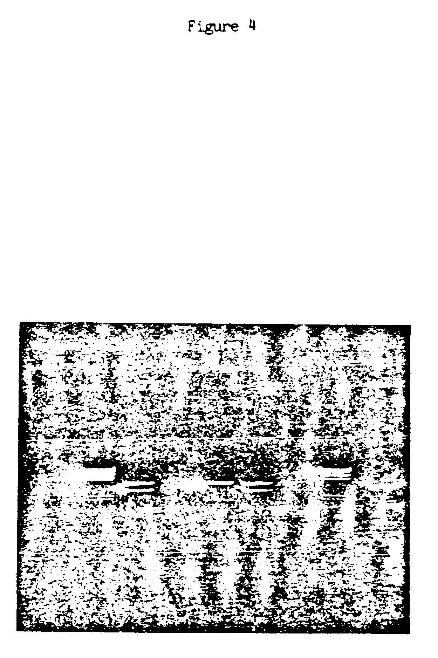

Einzelne ampicillinresistente Kolonien wurden auf tetracyclinhaltige Agarplatten ausgestrichen. Für ungefähr 5 % der getesteten Kolonien wurde Sensitivität für Tetracyclin festgestellt. Diese wurden zum Inokulieren von 2 ml ampicillinhaltigen LB-Mediums verwendet und über Nacht bei 37°C in einem Schüttler mit 220 upm inkubiert. Am nächsten Tag wurde eine Plasmid-Schnellpräparation nach einem Standard-Verfahren (Birnboim und Doly, Nucleic Acids Research 7, 1195-1204, 1979) durchgeführt. Die Plasmide wurden mit dem Restriktionsenzym SmaI verdaut und auf 1 %-igen Agarosegelen analysiert (Figur 4). Auf diese Weise wurden 21 Plasmide identifiziert, die Inserts im Größenbereich von 2000 - 3500 bp enthielten.

### Beispiel 8:

### Vorläufiges Charakterisieren der Inserts

Inserthaltige Plasmide wurden im großen Maßstab nach einem Standardverfahren hergestellt (Maniatis et al., Molecular Cloning, CSH, 1982) und durch Ethidiumbromiddichtegradientenzentrifugation gereinigt. Ungefähr 500 ng der Plasmid-DNAs wurden mit einer Anzahl von Restriktionsenzymen gespalten und auf 1 %-igen Agarosegelen analysiert. So wurden charakteristische Bandenmuster für jedes Plasmid erhalten, was eine erste Voraussage erlaubte, welche Inserts überlappende bzw. verschiedene Sequenzbereiche enthalten könnten.

### Beispiel 9:

### Sequenzanalyse des Inserts von Klon A5

Eines der inserthaltigen Plasmide, genannt Plasmid A5, wurde mit SmaI gespalten und auf einem 0,7 %-igen Agarosegel aufgetrennt. Die Bande, die das gesamte Insert darstellt, wurde aus dem Gel ausgeschnitten und die DNA wurde durch Elektroelution aus der Agarose gereinigt. Das isolierte Fragment wurde in veschiedenen Reaktionen mit häufig schneidenden Restriktionsenzymen, die glatte Enden erzeugen, verdaut, wie z.B. RsaI, AluI und HaeIII. Die doppelsträngige Form des Bakteriophagen M13mp8 wurde mit SmaI gespalten und dephosphoryliert. Die Mischungen der DNA-Fragmente wurden deproteinisiert, präzipitiert und mit dem M13-Vektor wie zuvor beschrieben ligiert. Die Transformation von E. coli JM105 Zellen, Identifizierung von rekombinanten Phagen durch das Farbselektionsverfahren und die Herstellung von einzelsträngiger DNA wurde nach Standardverfahren durchgeführt. Einzelne Klone wurden ausgewählt und nach dem Didesoxysequenzierungsverfahren, wie beschrieben von Sanger et al. (Proc.Natl.Acad.Sci. U.S.A. 74, 5463-5467, 1977), sequenziert. Als Primer wurde im allgemeinen der kommerziell erhältliche M13- Universalprimer benutzt. So wurde die Sequenz einer großen Anzahl verschiedener Fragmente des Klones A5 bestimmt, die schließlich zur Gesamt-DNA-Sequenz dieses Klones führte. Durch Vergleich dieser Sequenz mit publizierten Genomsequenzen verschiedener Flaviviren wurde festgestellt, daß A5 den Bereich des viralen Genomes enthält, der für die Strukturproteine C, M und E mit Ausnahme der aminoterminalen Hälfte des C-Proteines codiert. Die genaue Lokalisierung von A5 ist in Figur 1 dargestellt.

### Beispiel 10:

### cDNA-Synthese unter Verwendung eines FSME-Virus spezifischen Primers

Ein FSME-Virus spezifischer Primer wurde verwendet, um einen cDNA-Klon zu konstruieren, der den gesamten 5ʹ-Bereich des viralen Genoms einschließlich der gesamten Sequenzinformation des C-Proteins sowie das meiste des nicht-translatierten 5ʹ-Leaderbereichs enthält. Dieser Oligonukleotidprimer bestand aus 14 Nukleotiden, die komplementär zu einem Bereich des FSME-Genomes, der für die C-terminalen Aminosäuren des E-Proteines codiert, ist. Die Herstellung von viraler RNA und die Synthese doppelsträngiger cDNA wurden im wesentlichen wie oben beschrieben durchgeführt, mit der Ausnahme, daß dieses Mal nur 1 ug RNA und 0,5 ug des synthetischen Oligonukleotidprimers P1 verwendet wurden. Schließlich wurden die Inkubationszeiten für die Zweitstrangsynthese und die T4 DNA-Polymerasebehandlung auf 1 h bzw. 3 min reduziert.

### Beispiel 11:

### Klonieren in den Bluescript Vektor unter Verwendung von EcoRI-Linkern

Ausgehend von den zu diesem Zeitpunkt der Forschung vorhandenen Informationen konnte angenommen werden, daß der gesamte strukturelle Bereich keine EcoRI-Erkennungsstelle enthielt. Daher konnten synthetische EcoRI-Linker (New England Biolabs) für das Klonierverfahren verwendet werden. Ungefähr 1 ug cDNA wurde mit 1 ug Linker-DNA 6 h bei 14°C ligiert unter Verwendung von T4 DNA Ligase und einem Puffersystem, wie es oben beschrieben worden ist. Nach der Reinigung der Mischung durch Phenol- und Chloroformextraktionen und Ethanolpräzipitation wurde die DNA in 40 ul eines "medium salt" Restriktionsenzympuffers (CSH-Handbuch) resuspendiert und für 3 h mit einer überschüssigen Menge von EcoRI (80 Einheiten) bei 37°C verdaut. Anschließend wurde die Mischung auf einem 1%-igen Agarosegel aufgetrennt und cDNA in Größenbereichen von 1500 - 2500 bp wurde aus dem Gel durch Elektroelution wiedergewonnen.

Gleichzeitig wurde der neue Vielzweckvektor Bluescript KS M13+ (Stratagene) mit EcoRI gespalten. 100 ng dieses linearisierten Plasmides wurden mit der gesamten cDNA-Präparation gemischt und die DNAs wurden mit Ethanol ko-präzipitiert. Die Plasmid-cDNA Ligation wurde wie oben beschrieben durchgeführt. Ligationsmischungen wurden stufenweise von zweifach bis einhundertfach verdünnt und zur Transformation von E. coli XL-1 blue verwendet. Die Transformation und Identifikation von inserthaltigen Plasmiden durch Farbselektion wurde genau entsprechend den Instruktionen des Herstellers (Stratagene) durchgeführt. So wurden 4 Plasmide erhalten, die Inserts von annähernd 2500 bp Länge enthalten.

### Beispiel 12:

### Sequenzbestimmung des Klones P1-1

Eines der inserthaltigen Plasmide, genannt Plasmid P1-1, wurde zum Herstellen von einzelsträngiger Matrizen-DNA verwendet. Dies wurde durch Zufügen des Helferphagen R408 zu einer exponentiell wachsenden, Plasmid P1-1 enthaltenden Bakterienkultur in einer M.O.I. von 20:1 durchgeführt. Nach 8-stündiger Inkubation bei 37°C in einem Schüttler bei 240 upm wurde der Phagenpartikel enthaltende überstand geerntet und einzelsträngige P1-1-DNA wurde nach Standardverfahren hergestellt. Abweichend vom klassischen M13-System erlaubt das Bluescript-System die Herstellung von einzelsträngiger Matrizen-DNA, die die gesamte cDNA-Sequenz enthält, und macht so die zeitraubenden Subklonierungsverfahren überflüssig. Die Sequenzbestimmung von P1-1 wurde mit der Standard-Didesoxymethode unter Verwendung sowohl des Universal-M13- und synthetischer Oligonukleotidprimer, die verschiedenen viralen Sequenzen entsprechen, als Primer durchgeführt. So wurde gefunden, daß P1-1 die gesamte für Strukturproteine codierende Region überspannt und 120 bp über das Startcodon des C-Proteines hinausreicht. Die exakte Lokalisierung von P1-1 ist in Figur 1 ebenso dargestellt.

### Beispiel 13:

### Sequenzbestimmung verschiedener natürlicher Isolate durch direktes Sequenzieren der RNA.

Die für die Strukturproteine codierenden Sequenzen von verschiedenen natürlichen Isolaten des westlichen Subtyps des FSME-Virus wurden durch direkte Sequenzierung genomischer RNAs mit einer Modifikation der Sanger-Didesoxymethode, die im wesentlichen von Zimmern und Kaesberg (Proc.Natl.Acad.Sci. U.S.A. 75, 4257-4261, 1978) beschrieben wurde, durchgeführt. Verschiedene synthetische Oligonucleotide, die verschiedenen Stellen des Genomes entsprechen, wurden als Primer verwendet. Annähernd 1 ug genomischer RNA und 0,5 ug des jeweiligen Primers wurden in einem Volumen von 10 ul gemischt, 3 min auf 65°C erhitzt und dann langsam auf 42°C abgekühlt. Die Sequenzierungsreaktionen wurden durch Reverse Transkriptase (Amersham) 15 min bei 42°C katalysiert. Als Kontrolle wurde das FSME-Virus Prototyp Neudörfl gleichzeitig sequenziert und auf dem gleichen Polyacrylamidgel analysiert. Dies erlaubte eine einfache und zweifelsfreie Identifizierung von Sequenzvariationen in verschiedenen natürlichen Isolaten. Sequenzabweichungen wurden an Positionen, wie beschrieben im allgemeinen Teil der Beschreibung, gefunden.

### Beispiel 14:

### Aminoterminale Sequenzanalyse viraler Proteine

1 mg gereinigten westlichen Subtyps des FSME-Virus wurde einer SDS-PAGE nach Laemmli und Favre (J.Mol.Biol. 80, 575-599, 1973) auf 10 %-igen Acrylamidgelen unterworfen. Das E-Protein wurde lokalisiert, indem ein Filterpapierblot des Geles mit Amidoschwarz gefärbt wurde, das Protein aus dem Gel ausgeschnitten wurde und unter Verwendung einer ISCO-Elutionsapparatur 5 h bei 3 W elektroeluiert wurde. Die N-terminale Sequenzanalyse wurde nach dem von Chang et al. beschriebenen Verfahren (FEBS Letters 93, 205-214, 1978) durchgeführt. Die erhaltene Sequenz war Ser-Arg-Cys, und erlaubte daher das Feststellen der genauen Position des E-Proteins in der in Figur 3 gezeigten Sequenz.

Alternativ wurden Proteinkomplexe, die sowohl E als auch M enthielten, durch Solubilisieren von gereinigten FSME-Viren des westlichen Subtyps mit Triton X 100, gefolgt von Dichtegradientenzentrifugation in detergenzfreien Dichtegradienten, wie von Heinz und Kunz (J.Gen.Virol. 49, 125-132, 1980) beschrieben, hergestellt. Wie erwartet, ergab die aminoterminale Sequenzanalyse eine doppelte Sequenz, und zwar
Ser-Arg-Cys
Ser-Val-Leu.

Da es bereits bekannt war, daß Ser-Arg-Cys vom E-Protein stammte, wurde die Sequenz Ser-Val-Leu so als der Aminoterminus von M identifiziert (Fig. 3).

### Beispiel 15:

### Herstellung von Insertionsplasmiden, die fremde Gene durch homologe Rekombination in das Vaccinia Hind III J-Fragment durch Klonieren von 5'-Ende-Sequenzen der klonierten cDNA des FSME-Virus enthalten

In Figur 5 ist ein Plasmid A5 dargestellt, das auf eine dem Fachmann bekannte Weise als pBR322-abgeleitetes Plasmid hergestellt wurde. Wie in Figur 5 dargestellt, enthält das Plasmid A5 diejenige DNA-Sequenz, die für das Protein E des FSME-Virus codiert. In Figur 5 sind weiterhin die Restriktionsendonuklease-Schnittstellen ausgewählter Unterfragmente dargestellt, wobei die für das FSME-Protein E codierenden Regionen bezeichnet sind. Mit dem Pfeil ist die Transkriptionsrichtung in der genomischen RNA dargestellt.

Weiteres Virus- und Plasmid-Material sowie die Methoden, die angewandt wurden, um Plasmide herzustellen, die konkrete DNA-Teilabschnitte enthalten, die für das Protein E codieren, werden nachfolgend beschrieben:

### 15.1. Virus- und Zell-Kultur

Ein Vaccinia-Virus Wildtyp Stamm WR kann von der Hinterlegungsstelle ATCC unter der Nummer VR-119 erhalten werden. Menschliche TK⁻143 Zellen, die keine Thymidinkinase herstellen, sind allgemein erhältlich und beim Institut Pasteur hinterlegt (Hinterlegungsnr. I-732). Das Vaccinia-Virus wird in Vero-Zellen etwa zwischen den Passagen 47 bis 55 vermehrt. Die Zellen werden in MEM (minimal essential medium) mit 5% FCS (fetal calf serum), Antibiotika und Glutamin gezüchtet. Derjenige Virusstamm, der für die Rekombination verwendet werden soll, wird einmal in HAT (Hypoxanthin, Aminopterin, Thymidin)-Medium, enthaltend 5% FCS, passagiert, um ein TK⁺ Wildtyp-Virus zu selektieren. Rekombinantes TK⁻-Virus wird auf menschlichen TK⁻143-Zellen bestimmt, die in MEM mit 5% FCS, enthaltend 25 ug/ml BUdR, gewachsen sind. Die Rekombinanten können gefunden werden, indem die Zell-Monolayer mit niedrig schmelzender Agarose, enthaltend MEM, 5% FCS, 25 ug/ml BUdR sowie 250 ug/ml X-gal, überschichtet werden. Die Zellkultur wird 48 bis 72 Stunden in einem Inkubator bei 37°C gehalten. Die Menge der spontanen TK⁻-Virusmutanten wird durch Infizieren von TK⁻-Zell-Monolayern, in Anwesenheit oder Abwesenheit von 25 ug/ml BUdR bestimmt. Üblicherweise kann unter 10⁴ Viren eine Mutante beobachtet werden (Mackett et al. in "DNA cloning Vol. II, a practical approach", IRL Press, D.M.Glover (ed.) 1985).

### 15.2. Plasmide und Klonierungsverfahren

Das Plasmid A5, wie in Figur 5 dargestellt, das ein pBR322 abgeleitetes Plasmid ist, weist die gezeigte Restriktionsendonuklease-Karte auf.

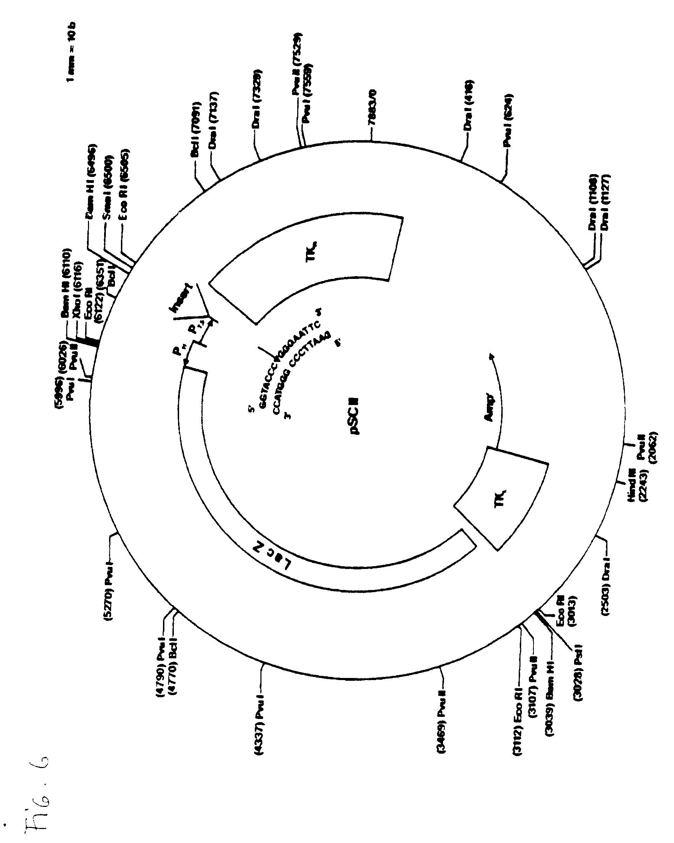

In Figur 6 ist das Plasmid pSC11 gezeigt, das das E. coli lac Z Gen für "blaue Farbe"-Bestimmung, die TK⁻-flankierenden Sequenzen und einen Vaccinia-Promotor enthält. Das Plasmid pSC11 ist bekannt und allgemein erhältlich, insbesondere jedoch durch das National Institute of Health zu beziehen.

Sämtliche Restriktionsendonukleasen, die für die Modifizierung der DNA verwendet wurden, wurden entsprechend den Gebrauchsanweisungen der Hersteller angewendet. Die Southern Blot Analyse und Isolierung der Plasmid-DNA wurde entsprechend den Standardverfahren durchgeführt (Maniatis et al., Molecular Cloning, CSH 1982).

Radioaktiv markierte Proben wurden entweder durch "nick repair"-Verfahren oder durch das pSP6-"Riboprobe"-Verfahren erhalten (Green et al., Cell 32, 681-694, 1983).

Die "Riboprobe"-Sonde, die für die vorliegende Erfindung verwendet wurde, wurde durch Ligieren einer internen FSME-Nukleotidsequenz von etwa 1,0 Kb in das Plasmid pSP64 FSME-Nukleotidsequenz von etwa 1,0 Kb in das Plasmid pSP64 hergestellt. Ehe die RNA-Synthese initiiert wurde, wurde das Plasmid mit EcoRI linearisiert. Ein typischer Assay bestand aus:

| | |
|---|---|
| Tris HCl 7,5 | 40 mM |
| MgCl₂ | 6 mM |
| Spermidin | 2 mM |
| NaCl | 20 mM |
| DTT | 10 mM |
| ATP, CTP, GTP jedes | 0,5 mM |
| Menschlicher plazentaler RNAse Inhibitor | 20 U |
| pSP64-P5 X EcoRI | 2 ug |
| α³²P UTP 400 Ci/mmol | 100 uCi |
| SP6 RNA Polymerase | 4 U |
| zu | 20 ul Gesamtvolumen |
| Inkubation: 1 h bei 40°C. | |

Die Nukleotide, die nicht eingebaut wurden wurden aus der rakioaktiv-markierten cRNA durch Säulenchromatografie getrennt.

### 15.3 In vivo Rekombinationsassay

### Infektion und Transfektion:

Rekombinationen wurden im wesentlichen wie bei Mackett et al. (s. 15.1) beschrieben, durchgeführt. Neben menschlichen TK⁻-Zellen, konnten auch Vero-Zellen oder Hühnerembryo-Fibroblastenzellen als Wirtszellen verwendet Werden. Etwa 1-3 Stunden vor der Transfektion wurden die Zellen mit einer Multiplizität der Infektion (m.o.i.) von zwischen 0,01 - 0,05 pfu/Zelle mit einem Wildtyp WR-Stamm infiziert. Die HEPES-gepufferte Saline für die Transfektion der DNA wurde folgendermaßen hergestellt: 10 x HBS, enthaltend 8,18 % NaCl (w/v); 5,94 % HEPES (w/v) sowie 0,2 % Na₂HPO₄ (w/v) wurden auf 2 x HBS vor der Verwendung verdünnt. Der pH-Wert wurde mit 1N NaOH auf exakt 7,05 justiert. Der Puffer wurde auf 1 x HBS verdünnt und durch Filtrieren sterilisiert. Zu 1 ml dieses 1 x HBS wurde eine Lösung von 1 bis 5 ug Plasmid-DNA und 10 bis 20 ug Carrier-DNA (entweder Wildtyp des Vaccinia-Virus oder Lachsspermien-DNA) zugegeben. Insbesondere die Zugabe der Carrier-DNA schien wesentlich zu sein. Die beschriebene Mischung wurde gründlich 1 min lang auf einem Vortex-Schüttler gemischt. Danach wurden langsam 2 M CaCl₂-Lösung bis zu einer Endkonzentration von 0,125 M zugegeben. Ein DNA-Präzipitat wurde nach 20 bis 30 min bei 25°C sichtbar. Das Medium wurde von den infizierten Zellen entfernt, der Monolayer einmal mit dem Zellkulturmedium gewaschen und nach dem Entfernen der gesamten Restflüssigkeit wurde das DNA-Präzipitat langsam zugegeben. 30 min später wurde bei 25°C frisches Medium zugegeben und die Zellen bei 37°C inkubiert. Nach 3 bis 4 h wurde das Medium wieder entfernt und gegen frisches Zellkulturmedium ausgetauscht. Die Zellen wurden üblicherwese nach 24 oder 48 h durch Abschaben geerntet, bei niedriger Geschwindigkeit sedimentiert und das Zellpellet wurde in einem Gesamtvolumen von 500 ul Medium gesammelt.

### Plaque-Assay und Reinigung rekombinanter Viren:

Die Virussuspension wurde dreimal gefroren und wieder aufgetaut; 60 ul der Suspension werden mit 1/5 Volumen von 1,25 % Trypsin 30 min bei 37°C inkubiert. Eine Serie von drei Verdünnungen (1:10) wurde zu einem konfluenten Monolayer von TK⁻143-Zellen, die in 10 cm²-Platten gewachsen sind, zugegeben. Nach 30 bis 60 minütigem Inkubieren bei 37°C wurde das Medium entfernt, sodann wurden 3 ml des Mediums mit 25 ug/ml Bromdesoxyuridin zugegeben. Nach 24 oder 48 h bei 37°C wurde das Medium wieder entfernt und durch eine Agarose-Überschichtung, enthaltend 25 ug/ml Bromdesoxyuridin und 125 ug/ml X-gal ersetzt. Nach weiteren 24 bis 48 h Inkubation konnten blaue Plaques festgestellt werden. Einzelne dieser Plaques wurden ausgestochen und in 500 ul Medium suspendiert. Diese virusenthaltende Suspension wurde, wie oben beschrieben, mit Trypsin behandelt. Plaque-Reinigungen wurden mindestens zweimal durchgeführt.

### 15.4 Herstellung und Charakterisierung sowohl von Wildtyp- als auch rekombinanter Vaccinia-DNA

Von 175 cm²-Flaschen wurden infizierte Zellen abgeschabt und zweimal mit PBS-Ca²⁺/Mg²⁺ bei einem pH-Wert von 7,4 gewaschen. Nach Zentrifugation wurden die Zellen in 250 ul PBS resuspendiert. Die Zellen wurden dreimal kurzzeitig gefroren und wieder aufgetaut. Ein Volumen von 250 ul eines 2 x Zell-Lysis Puffer (1 % Triton X 100; 70 mM β-Mercaptoethanol; 40 mM EDTA) wurde zugegeben und die Zellen wurden 5 min auf Eis lysiert. Die Kerne wurden 1 min lang bei 16000 x g vom Cytoplasma getrennt. Der Überstand, der aggregierte Viruspartikel enthielt, wurde in ein neues Röhrchen überführt und 30 min bei 16000 x g zentrifugiert, um das Virus zu pelletieren. Sodann wurde Virus-Lysis-Puffer (10 mM Tris-HCl pH 8; 1 mM EDTA; 5 mM β-Mercaptoethanol; 200 mM NaCl; 1 % SDS; 150 ug/ml Proteinase K) in einer Menge von 100 ul zu dem Pellet zugegeben und 30 min bei 56°C inkubiert. Die Nukleinsäuren wurden sodann 3 x mit Tris-HCl gesättigtem Phenol extrahiert, sodann mit Chloroform:Isoamylalcohol (24:1) reextrahiert. Es wurde darauf geachtet, daß die DNA keinen Scherkräften ausgesetzt wurde. Die Nukleinsäure wurde mit 0,1 M NaCl und 2,5-fachem Volumen Ethanol bei -20°C übernacht präzipitiert. Etwa 10 bis 20 ug der DNA konnten aus 10⁷ Zellen isoliert werden. Nach dem Zentrifugieren (30 min bei 17900 x g) wurde die DNA in einem kleinen Volumen von 20 bis 40 ul H₂O gelöst. Die DNA wurde mit der Restriktionsendonuklease Hind III geschnitten und die Fragmente auf 0,6 %-igen Agarosegelen in TA-Puffer getrennt (Rice et al., J.Virol. 56, 227-239, 1985).

### 15.5. Auffinden von Nukleinsäuren durch Hybridisierung

### Southern Blot

Nach der Übertragung der DNA aus den Gelen (30', 0,5N NaOH und 2 x 10', 10 x SSC/0,5 M Tris HCl pH 7,5) auf Nitrocellulose-Filter wurden diese bei 80°C 4 h lang gebacken. Die Filter wurden 6 h bei 65°C in 5 x Denhardtlösung (1 x Denhardt = 0,02 % Ficoll, 0,02 % Polyvinylpyrrolidon, 0,02 % BSA) vorhybridisiert. Die nassen Filter wurden in Plastikbeuteln versiegelt und mit der Hybridisierungslösung sowie der radioaktiven Sonde 18 h bei 43° C inkubiert. Die Filter wurden bei 65° C dreimal jeweils 30' jeweils in 2 x SSC, 1 x SSC und 0,1 x SSC mit 0,1 % SDS und 20 mM NaPO₄ gewaschen. Nach dem Lufttrocknen der Filter wurden diese Kodak X-omat AR X-ray Filmen ausgesetzt.

Ein "Riboprobe"-Vektor (pSP6), der ein FSME-Fragment aus dem in Figur 5, gezeigten Plasmid A5 enthielt, wurde zur Herstellung einer FSME Virus spezifischen "Riboprobe" verwendet. Radioactiv markierte cRNA wurde mit einer Hybridisierungslösung (50 % Formamid, Amberlite gereinigt; 5x SSC, 1 x Denhardtlösung, 0,1 % SDS; 100 ug/ml Heringsperma DNA) gemischt. Das gesamte Volumen der Hybridisierungslösung wurde bestimmt, da 50 ul für 100 ul/cm²-Filter für optimale Hybridisierungsbedingungen nötig sind. Die Menge der radioaktiven cRNA war etwa 10⁷ cpm/ml oder etwa 10⁶ cpm/cm²-Filter. Die Lösung wurde 5ʹ bei 65° C erhitzt.

### Spot Blot

Diese Experimente wurden nach Mackett et al. (s.15.1) mit leichten Modifikationen durchgeführt. Material von 1/4 der isolierten Plaques wurde verwendet, um eine 35 mm Petrischale zu infizieren. 48 h nach Infektion wurden die Zellen geerntet und in einem Puffer gewaschen, der 50 mM Tris pH 7,5 und 100 mM NaCl enthielt. Schließlich wurden die Zellen in 500 ul dieses Puffers resuspendiert. 100 ul Aliquots wurden in Vacuum auf Nitrocellulose gesaugt. Nach dem Trockenen wurden die Filter, eingeweicht mit 0,5 M NaOH 3ʹ lang auf Chromatographiepapier gegeben. Danach wurden die Filter auf Papierfiltern, die mit 2 x SSC/1 M Tis-HCl pH 7,5 jeweils 3ʹ lang getränkt worden waren, neutralisiert. Die Filter wurden sodann mindestens 2 h gebacken und im Weiteren so behandelt, wie oben für die Southern Blot Hybridisierung beschrieben.

### 15.6 Proteinanalyse

Vero-Zellen oder TK⁻143-Zellen wurden mit einer m.o.i. von 1 pfu/Zelle in einem Gesamtvolumen von etwa 1 bis 2 ml/25 cm²-Flaschen infiziert. In einigen Experimenten wurden die Zellen mit 100 uCi ³⁵S-Methionin/ml markiert. Wenn der CPE stark war, wurden die Zellen geerntet, zweimal in PBS gewaschen und schließlich in 100 ul RIPA-Puffer (150 mM NaCl; 10 mM Tris-HCl pH 7,2; 1 % Natriumdesoxycholat; 1 % Triton x 100; 0,1 % SDS; 100 ug/ml PMSF) resuspendiert (50-150 ul für 5 x 10⁵ - 1,5 x 10⁶ Zellen). Nach 10 min auf Eis wurden die Proben 30 sek lang beschallt. Zu 8 ul dieser Proteinmischung wurden 12 ul eines 2 x Kochpuffers (0,2 M DTT; 4 % SDS; 0,16 M Tris-HCl pH 6,8; 20 % Glycerin; 1/100 Volumen von 5 % BPB in Ethanol) zugegeben; die Proteine wurden 10 min lang bei 100° C denaturiert und einer Elektrophorese auf 15 % SDS-Poyacrylamid-Gelen unterworfen (Bodemer et al., Virology 103, 340-349, 1980). Die Proteine wurden aus dem Gel auf Nitrocellulose-Filter in einem Puffer, enthaltend 0,192 M Glycin, 0,025 M Tris pH 8,3 und 20 % Methanol, bei einer Stromstärke von etwa 1,5 A 2,5 h lang übertragen. Die Nitrocellulose-Filter wurden sodann in einer Blockierungslösung "NET" (0,15 M NaCl, 5 mM EDTA, 50 mM Tris pH 7,4, 0,05 % Triton x 100, 2 % BSA) inkubiert. Menschliches Serum und ein Enzym-markierter zweiter gegen humane Immunglobuline gerichteter Antikörper wurde in NET bei einer Verdünnung von 1:500 zugegeben und jeweils 2 h inkubiert. Die Streifen wurden mit NET 3 x 10 min gewaschen und sodann mit TBS (20 mM Tris pH 7,5, 0,9 % NaCl, 2 % BSA) 3 x 10 min lang eingeweicht. Die Banden wurden in 50 ml TBS und dem chromogenen Substrat DAB und H₂O₂ entwickelt.

### 15.7. Herstellung der Insertionsplasmide

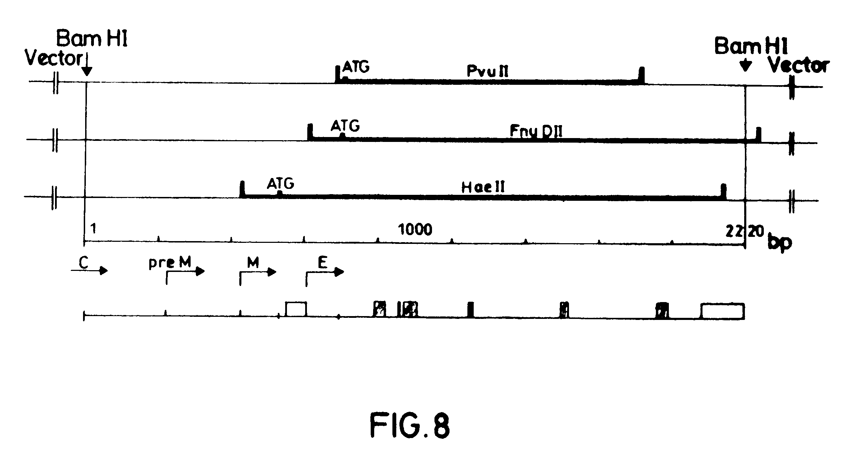

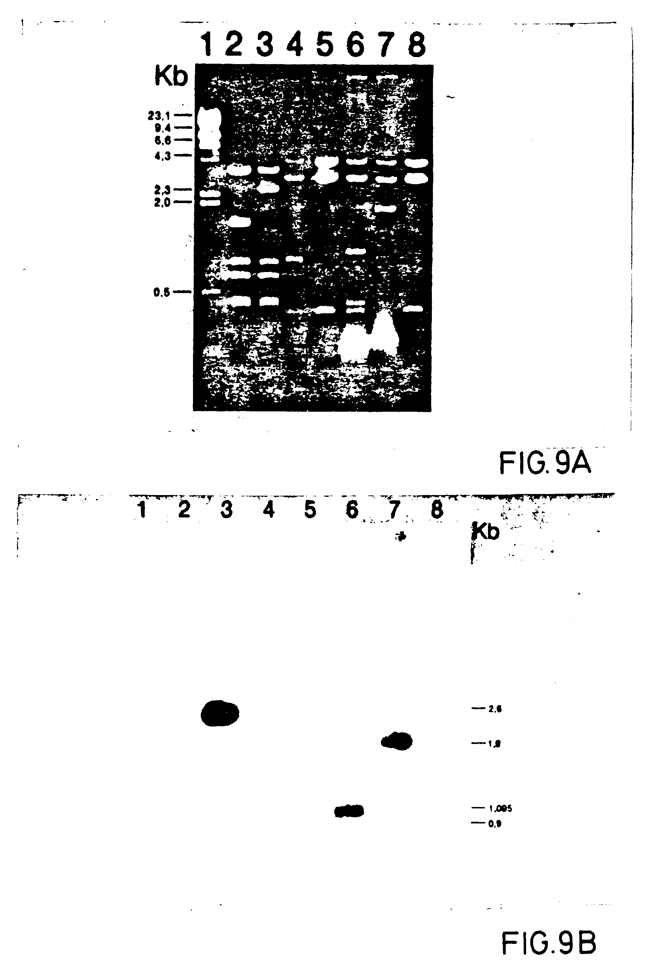

Mit den oben beschriebenen Materialien und Methoden wurden nunmehr Plasmide, die die DNA-Sequenzen des Protein E enthalten, hergestellt. Zuerst wurde das Plasmid A5 (Fig. 5) mit den Restriktionsendonukleasen PvuII, FnuDII und HaeII verdaut. Die Fragmente wurden in die einzige SmaI -Stelle des Plasmids pSC11 (Figur 6) inseriert. Die drei FSME-Fragmente wurden aus Agarosegelen durch Elektroelution isoliert und mit dem Plasmid pSC11 ligiert, wobei letzteres ebenfalls an der einzigen SmaI-Stelle linearisiert wurde. Die physikalische Karte dieser Plasmide ist in Figur 7 gezeigt. Die drei Fragmente haben eine Größe von 1022 bp (PvuII), 1485 bp (FnuDII) und 1553 bp (HaeII). Die genannten drei Restriktionsendonukleasen wurden verwendet, da die Nukleotidsequenz, die für den inneren Teil des Protein E codiert, sowohl mit als auch ohne hydrophobe Signale oder Membranankersequenzen durch einen Hydropathie-Plot, durchgeführt mit einem IBI Pustell Computerprogramm, gefunden worden waren (Figur 8). Die Orientierung der inserierten Protein E-Sequenzen wurden mit Bezug auf den p 7.5 E/l Vaccinia-Promotor durch Southern Blot Analyse bestimmt. Ein Schneiden des pSC11-P6 Plasmids mit PvuI oder BamHI ergab klar ein 2,585 Kb- oder ein 0,919 Kb Fragment (Figur 9A Reihen 3 und 4), das jeweils mit einer ³²P-markierten "Riboprobe"-Sonde hybridisierte (Figur 9B, Reihe 3 und 4). Das Plasmid pSC11-F41 konnte mit BamHI oder EcoRI in Fragmente geschnitten werden; Fragmente mit einer Größe von 1,019 Kb oder 1,879 Kb hybridisierten jeweils ausschließlich mit der "Riboprobe"-Sonde (Figur 9B, Reihen 6 und 7).

### 15.8. In vivo Rekombination mit FSME-Insertionsplasmiden und Wildtyp Vaccinia-Virus

Vero-Zellen Monolayer, die mit dem Vaccinia-Stamm WR infiziert worden waren, wurden mit Plasmid DNA transfiziert. Nach 48-72 h wurde ein starker cytopathogener Effekt sichtbar und die Zellen wurden geerntet. Das Virus wurde aus den Zellen durch wiederholtes Einfrieren und Auftauen sowie eine Trypsin-Behandlung wie beschrieben entlassen. Sodann wurden TK⁻-Zellen-Monolayer mit dieser Virussuspension infiziert. Die Zellen wuchsen in Anwesenheit von BUdR. Sich entwickelnde Plaques wurden unter einer Agaroseüberschichtung, enthaltend das chromogene Substrat X-gal, sichtbar gemacht. Virusverdünnungen wurden durchgeführt, so daß sich 10 - 30 Plaques pro 35 mm - Platten ergaben. Einzelne blaue Plaques wurden ausgestochen und für die weitere Infektion von TK⁻-Zellen zum Zwecke einer weiteren Plaque-Reinigung der Infektionsselektion verwendet, oder von Vero-Zellen für die Analyse viraler Makramoleküle.

### 15.9. Spot Blot Analyse zum Auffinden Rekombinanter Viren

Kleine Proben (etwa 100 ul) infizierter Zell-Monolayer aus 35 mm - Platten oder 25 cm² Kulturflaschen wurden aufgeteilt und an ein "nick-repair"-markiertes pSC11-Plasmid (das keine FSME-Sequenzen enthielt) oder an die FSME-"Riboprobe" (Figur 10) hybridisiert. Mock-infizierte Zellen zeigten mit beiden Sonden keinerlei Signal. Das Virus vSC8, das das lac Z Gen und TK-Vaccinia-Sequenzen, jedoch keine FSME-Sequenzen enthielt, zeigte mit der FSME-"Riboprobe" ein negatives Ergebnis. Es waren jedoch, wie erwartet, alle Zellen mit Viren aus vier verschiedenen individuellen Plaque-Isolierungen positiv mit der FSME- und der pSC11-Sonde.

### 15.10. Analyse der DNA aus Rekombinanten Viren

Vero-Zellen Monolayer wurden mit einer m.o.i. von 1 - 10 pfu/Zelle mit plaquegereinigten, vermehrten Vaccinia-Rekombinanten infiziert. Die Virus-DNA wurde aus Viruspartikeln, die aus infizierten Zellen entlassen wurden, isoliert und durch Resstriktionsendonuklease-Verdauung sowie Southern Blot Hybridisierung analysiert. Das 5 kb Hind III J Fragment der Wildtyp-DNA (wie in Figur 11, Reihe 6 gezeigt) wurde durch ein größeres Hind III - Fragment, bestehend aus dem ursprünglichen Hind III J - Fragment und denjenigen DNA-Sequenzen, die durch in vivo Rekombination in den TK-Genlokus inseriert worden waren, ersetzt. Das vSC8 - Virus enthielt ein 3,5 Kb lac Z Gen, das Hind III J - Fragment wanderte daher bei einer Größe von etwa 8,5 Kb (Figur 11, Reihe 5). Da größere Fragmente sogar in niedrigprozentigen Gelen nicht ohne weiteres getrennt wurden können, wurde eine abschließende Bestimmung der rekombinanten Virus-DNA durch Southern Blot Hybridisierung durchgeführt (Figur 12). Mit ³²P-markierten Sonden konnten ganz klar Fragmente aufgefunden werden, die die durch die Klonierungsstrategie vorausgesagten Größen aufwiesen. Das rekombinante Virus, das durch Rekombination von pSC11-P6 gebildet wurde, hatte ein geringfügig abweichendes (etwa 500 Bp), kleineres Hind III - Fragment als die "pSC11-F41" Rekombinante (Figuren 12A und B). DNAs aus nicht infizierten Zellen zeigten weder Hybridisierung mit der pSC11 "nick-repair" markierten Sonde noch mit der "Riboprobe" (Figuren 12A und B, Reihe 9). Die DNA aus dem Virus vSC8 hybridisierte nur an die pSC11 "nick-repair" markierte Sonde aufgrund der gemeinsamen TK-Sequenz und des lac Z Gens (Figur 12B, Reihe 8). Die FSME-"Riboprobe" zeigte wie erwartet keine Hybridisierung. Sowohl die "Riboprobe" als auch die "nick-repair" markierte Sonde identifizierten jedoch "rekombinante" Hind III J - Fragmente unterschiedlicher Größe (Figuren 12 A und B, Reihen 1-3 und 7). Die DNA des Vaccinia-Virus, das nach Rekombination mit dem Insertionsplasmid pSC11 und Wildtyp-Virus entstanden war, ist in den Figuren 12 A und B, Reihen 4 - 6 gezeigt. Mit der "Riboprobe" (Figur 12 A) wurde keine Hybridisierung beobachtet, jedoch mit dem Plasmid pSC11 (Figur 12 B).

### 15.11 Proteinanalyse

Es wurden Zellen mit Wildtyp oder rekombinanten Viren mit einer m.o.i. zwischen 1 und 10 pfu/Zelle infiziert. Die Zellen wurden mit ³⁵S-Methionin nach verschiedenen Zeiten nach Infektion markiert, geerntet und nacheinander auf denaturierenden SDS Polyacrylamid-Gelen analysiert. Protein E-Varianten-Moleküle wurden durch Western Blot Analyse mit einem Human-Antiserum bestimmt. In Zellen, die mit dem rekombinanten Virus P6 infiziert worden waren, konnte ein FSME Virus spezifisches Protein mit einem Molekulargewicht von ungefähr 38000 Dalton nachgewiesen werden. Dieses Protein wies die aus der Primärsequenz abgeleitete Größe auf und besteht aus 335 Aminosäuren aus dem FSME-Protein E und 87 Aminosäuren des Vaccinia-TK-Gens. Da die FSME-Sequenz keine internen Stopcodons enthält, war diese Fusion zu erwarten. Mit einem zweiten rekombinanten Virus, das das FnuDII - Fragment der FSME-Protein E-Sequenz enthält, ist ein Protein von etwa 42 KD zu erwarten.

Der Zeitablauf der Synthese der FSME-Proteine in Zellen, die mit Vaccinia-Virus Rekombinanten infiziert wurden , wurde durch Markierung der Zellen zu unterschiedlichen Zeiten nach Infektion während einer kurzen Zeit (4 h) mit ³⁵S Methionin bestimmt. Da der p7.5-Promotor sowohl zu einer frühen als auch einer späten Phase des Infektionszyklus aktiv ist, war zu erwarten, daß zu einem frühen und einem späten Zeitpunkt nach Infektion die Zellen FSME-Proteine produzierten. Im Vergleich dazu reguliert der späte p11-Promotor die Synthese des β-gal-Proteins, das ein Molekulargewicht von 116 Kd hat. Dieses Protein erschien daher verhältnismäßig spät nach Infektion. Die geschilderten frühen oder späten Aktivitäten der Promotoren wirkten sich auf den Zeitpunkt des Erntens der Zellen aus, insbesondere wenn größere Mengen des Virus für die Reinigung und Herstellung der gewünschten Proteine zur Verfügung gestellt wurden mußten.

### Beispiel 16:

### Impfstoffherstellung

Für das Herstellen eines Impfstoffes kann beispielsweise ein erfindungsgemäß hergestelltes Antigen in einer reinen und wirksamen Form in einer Pufferlösung suspendiert und, um die immunogene Wirksamkeit zu verbessern, mit einem Adjuvans in einer dem Fachmann bekannten Weise gelöst werden.

## Patentansprüche (Patentansprüche für folgende(n) Vertragsstaat(en): AT, BE, CH, LI, DE, FR, GB, GR, IT, LU, NL, SE)

1. DNA-Molekül, **dadurch gekennzeichnet**, daß es mindestens eine vom westlichen Subtyp des FSME-Virus abgeleitete DNA-Sequenz umfaßt, die für ein oder mehrere Polypeptide aus der die Proteine C, prM, M oder E des westlichen Subtyps des FMSE-Virus umfassenden Gruppe kodiert, wobei das Polypeptid eine Aminosäuresequenz umfaßt, die in der in Figur 3 dargestellten Aminosäuresequenz enthalten ist.

2. DNA-Molekül nach Anspruch 1, **dadurch gekennzeichnet**, daß das Polypeptid den Bereich einer oder mehrerer antigenen Determinanten eines der Proteine aus der die Proteine C, prM, M oder E des westlichen Subtyps des FSME-Virus umfassenden Gruppe umfaßt.

3. DNA-Molekül nach Anspruch 1, **dadurch gekennzeichnet**, daß es die gesamte für die Strukturproteine des FSME-Virus kodierende DNA-Sequenz umfaßt.

4. DNA-Molekül nach Anspruch 3, **dadurch gekennzeichnet**, daß es die in Figur 1 dargestellte Sequenz umfaßt.

5. DNA-Molekül nach Anspruch 2, **dadurch gekennzeichnet**, daß es die für den Bereich einer antigenen Determinante des Proteins E kodierende DNA-Sequenz umfaßt.

6. DNA-Molekül nach mindestens einem der Ansprüche 1 bis 5, **dadurch gekennzeichnet**, daß es durch reverse Transkription mit Reverser Transkriptase von einem gereinigten RNA-Molekül des westlichen Subtyps des FSME-Virus erhalten worden ist.

7. DNA-Molekül nach mindestens einem der Ansprüche 1 bis 5, **dadurch gekennzeichnet**, daß es durch chemische DNA-Synthese hergestellt wird.

8. DNA-Molekül, **dadurch gekennzeichnet**, daß es mit einem DNA-Molekül nach einem der Ansprüche 1 bis 7 unter stringenten Bedingungen hybridisiert.

9. DNA-Molekül nach mindestens einem der Ansprüche 1 bis 8, **dadurch gekennzeichnet**, daß es DNA-Sequenzen umfaßt, die sich von den DNA-Molekülen nach den Ansprüchen 1 bis 8 als Folge von Degeneration des genetischen Codes und/oder Mutationen und/oder Transpositionen unterscheiden, aber noch für ein Polypeptid mit mindestens einer antigenen Determinante eines der Proteine kodieren, die aus der die Proteine C, prM, M oder E des westlichen Subtyps des FSME-Virus umfassenden Gruppe ausgewählt sind.

10. DNA-Molekül nach mindestens einem der Ansprüche 1 bis 9, **dadurch gekennzeichnet**, daß es mit zusätzlichen DNA-Sequenzen kombiniert ist.

11. DNA-Molekül nach Anspruch 10, **dadurch gekennzeichnet**, daß die zusätzlichen DNA-Sequenzen Replikation und Expression des DNA-Moleküls in einer Zellkultur erlauben und Sequenzen umfassen, die aus der Promotoren, Enhancer, Polyadenylierungssignale und Splicesignale umfassenden Gruppe ausgewählt sind.

12. RNA-Molekül, **dadurch gekennzeichnet**, daß es mindestens eine von der RNA des westlichen Subtyps des FSME-Virus abgeleitetete RNA-Sequenz umfaßt und für ein oder mehrere Polypeptide mit den in Figur 3 dargestellten Aminosäuresequenzen kodiert.

13. RNA-Molekül nach Anspruch 12, **dadurch gekennzeichnet**, daß es die gesamte RNA-Sequenz umfaßt, die für die Strukturproteine kodiert.

14. RNA-Molekül nach Anspruch 13, **dadurch gekennzeichnet**, daß es die in Figur 2 dargestellte Sequenz umfaßt.

15. RNA-Molekül nach Anspruch 12, **dadurch gekennzeichnet**, daß es für eines der Proteine kodiert, die aus der die Proteine C, prM, M oder E des westlichen Subtyps des FSME-Virus umfassenden Gruppe ausgewählt sind.

16. RNA-Molekül nach mindestens einem der Ansprüche 12 bis 15, **dadurch gekennzeichnet**, daß es für den Bereich mindestens einer antigenen Determinante eines der Proteine kodiert, die aus der die Proteine C, prM, M oder E des westlichen Subtyps des FSME-Virus umfassenden Gruppe ausgewählt sind.

17. RNA-Molekül nach Anspruch 15, **dadurch gekennzeichnet**, daß es für den Bereich mindestens einer antigenen Determinante des Proteines E kodierende RNA-Sequenzen umfaßt.

18. RNA-Molekül nach mindestens einem der Ansprüche 12 bis 17, **dadurch gekennzeichnet**, daß es durch Isolieren und Reinigen von RNA des westlichen Subtyps des FSME-Virus oder durch rekombinante RNA/DNA-Techniken erhalten wird.

19. RNA-Molekül nach Anspruch 18, **dadurch gekennzeichnet**, daß es durch Transkribieren isolierter und gereinigter Virus-RNA in DNA durch Reverse Transkriptase und anschließende Transkription dieser DNA wiederum in RNA erhalten wird.

20. RNA-Molekül **dadurch gekennzeichnet**, daß sein komplementärer Strang unter stringenten Bedingungen mit einem RNA-Molekül nach einem der Ansprüche 12 bis 18 hybridisiert.

21. Vektor, **dadurch gekennzeichnet**, daß er Insertionen von DNA-Sequenzen nach einem der Ansprüche 1 bis 11 oder RNA-Sequenzen nach einem der Ansprüche 12 bis 20 umfaßt, wobei der Vektor aus der Plasmide, Viren und Cosmide umfassenden Gruppe ausgewählt ist.

22. Vektor nach Anspruch 21, **dadurch gekennzeichnet**, daß er das Plasmid pBR322 ist, das eine DNA-Sequenz enthält, die für ein aus der die Proteine C, prM, M oder E des westlichen Subtyps des FSME-Virus oder Teile davon umfassenden Gruppe ausgewähltes Protein kodiert.

23. Vektor nach Anspruch 22, **dadurch gekennzeichnet**, daß er das bei der DSM mit der Hinterlegungsnummer DSM 4382 hinterlegte Plasmid A5, wie in Figur 5 dargestellt, ist.

24. Vektor nach Anspruch 22, **dadurch gekennzeichnet**, daß er das bei der DSM mit der Hinterlegungsnummer DSM 4383 hinterlegte Plasmid P1-1 ist.

25. Vektor nach mindestens einem der Ansprüche 21 oder 22, **dadurch gekennzeichnet**, daß er ein Insertionsplasmid ist, das über eine homologe Rekombination DNA-Sequenzen für das Protein E oder Teile davon in ein HindIII J-Fragment eines Vaccinia-Virus überträgt.

26. Vektor nach Anspruch 25, **dadurch gekennzeichnet**, daß er das Plasmid pSC11-H1 gemäß Figur 7 ist, das die Nukleotide 6503-8051 der für Protein E kodierenden DNA-Sequenz enthält.

27. Vektor nach Anspruch 25, **dadurch gekennzeichnet**, daß er das Plasmid pSC11-F41 gemäß Figur 7 ist, das die Nukleotide 6503-7998 der für Protein E kodierenden DNA-Sequenz enthält.

28. Vektor nach Anspruch 25, **dadurch gekennzeichnet**, daß er das Plasmid pSC11-P6 gemäß Figur 7 ist, das die Nukleotide 6503-7524 der für Protein E kodierenden DNA-Sequenz enthält.

29. Vaccinia-Expressions-Vektor, **dadurch gekennzeichnet**, daß er über homologe Rekombination DNA-Sequenzen für das Protein E enthält, bevorzugt inserierte DNA-Sequenzen aus einem der Plasmide gemäß einem der Ansprüche 25-28, und zur Expression von Protein E oder Teilpeptiden von Protein E, enthaltend die zu den genannten DNA-Sequenzen korrespondierenden Aminosäuresequenzen, fähig ist.

30. Vehikel, **dadurch gekennzeichnet**, daß es Insertionen von DNA-Sequenzen nach einem der Ansprüche 1-11 oder RNA-Sequenzen nach einem der Ansprüche 12-20 umfaßt, wobei das Vehikel ein Bakterium ist.

31. Vehikel nach Anspruch 30, **dadurch gekennzeichnet**, daß das Bakterium Salmonella typhi ist.

32. Zellkultur, **dadurch gekennzeichnet**, daß sie irgendeine der Sequenzen nach den Ansprüchen 1 bis 20 enthält.

33. Zellkultur nach Anspruch 32, **dadurch gekennzeichnet**, daß sie die Expression von Polypeptiden, die durch die RNA- oder DNA-Sequenzen nach einem der Ansprüche 1 bis 20 kodiert werden, erlaubt.

34. Zellkultur nach Anspruch 33, **dadurch gekennzeichnet**, daß die Zellkultur eine Säugetier-Zellkultur ist.

35. Zellkultur nach einem der Ansprüche 32 bis 34, **dadurch gekennzeichnet**, daß es eine Vero-Zellkultur oder eine Hühnerembryo-Fibroblastenzellkultur ist, die einen Vaccinia-Expressions-Vektor gemäß Anspruch 30 enthält und in der der den insertierten DNA-Sequenzen entsprechende Teil des Protein E exprimiert wird.

36. Peptid oder Polypeptid mit mindestens einer antigenen Determinante, wobei das Peptid oder Polypeptid eine Aminosäuresequenz umfaßt, die in der Aminosäuresequenz von Figur 3 enthalten ist.

37. Peptid oder Polypeptid nach Anspruch 36, **dadurch gekennzeichnet**, daß es durch Expression einer der Sequenzen nach einem der Ansprüche 1 bis 20 in einem geeigneten Expressionssystem hergestellt wird.

38. Peptid oder Polypeptid nach Anspruch 37, **dadurch gekennzeichnet**, daß es in einer Zellkultur gemäß Anspruch 35 hergestellt wird.

39. Peptid oder Polypeptid nach Anspruch 36, **dadurch gekennzeichnet**, daß es chemisch synthetisiert wird.

40. Zusammensetzung, **dadurch gekennzeichnet**, daß sie eines oder mehrere der Peptide oder Polypeptide nach einem der Ansprüche 36 bis 39 umfaßt.

41. Lebend-Impfstoff, **dadurch gekennzeichnet**, daß er DNA-Sequenzen nach einem der Ansprüche 1 bis 11 und/oder RNA-Sequenzen nach einem der Ansprüche 12 bis 20 umfaßt.

42. Lebend-Impfstoff, **dadurch gekennzeichnet**, daß eine oder mehrere der DNA-Sequenzen nach einem der Ansprüche 1 bis 11 mit Vaccinia-Virus DNA kombiniert sind.

43. Lebend-Impfstoff nach Anspruch 42, **dadurch gekennzeichnet**, daß er einen Vaccinia-Vektor gemäß Anspruch 29 enthält.

44. Impfstoff, **dadurch gekennzeichnet**, daß er eine Zusammensetzung nach Anspruch 40 enthält.

45. Verwendung eines der Impfstoffe nach den Ansprüchen 41 bis 44 zum Herstellen spezifischer Immunglobuline.

46. Diagnostisches Mittel, **dadurch gekennzeichnet**, daß es eine Zusammensetzung nach Anspruch 40 enthält.

47. DNA- oder RNA-Sonde, **dadurch gekennzeichnet**, daß sie unter strigenten Bedingungen mit DNA- oder RNA-Sequenzen nach einem der Ansprüche 1 bis 9 und 12 - 20 hybridisiert.

## Patentansprüche (Patentansprüche für folgende(n) Vertragsstaat(en): ES)

1. Verfahren zur Herstellung eines DNA-Moleküls das mindestens eine vom westlichen Subtyp des FSME-Virus abgeleitete DNA-Sequenz umfaßt, die für ein oder mehrere Polypeptide aus der die Proteine C, prM, M oder E des westlichen Subtyps des FSME-Virus umfassenden Gruppe kodiert, wobei das Polypeptid eine Aminosäuresequenz umfaßt, die in der in Figur 3 dargestellten Aminosäuresequenz enthalten ist, umfassend die Schritte
- Reinigen des RNA-Moleküls, und
- Transkription der RNA-Matrize in das DNA-Molekül mit Hilfe von Reverser Transkriptase; oder
- chemische Synthese des DNA-Moleküls.

2. Verfahren nach Anspruch 1, **dadurch gekennzeichnet**, daß das Polypeptid den Bereich einer oder mehrerer antigenen Determinanten eines der Proteine aus der die Proteine C, prM, M oder E des westlichen Subtyps des FSME-Virus umfassenden Gruppe umfaßt.

3. Verfahren nach Anspruch 1, **dadurch gekennzeichnet**, daß das DNA-Molekül die gesamte für die Strukturproteine des FSME-Virus kodierende DNA-Sequenz umfaßt.

4. Verfahren nach Anspruch 3, **dadurch gekennzeichnet**, daß das DNA-Molekül die in Figur 1 dargestellte Sequenz umfaßt.

5. Verfahren nach Anspruch 2, **dadurch gekennzeichnet**, daß das DNA-Molekül die für den Bereich einer antigenen Determinante des Proteins E kodierende DNA-Sequenz umfaßt.

6. Verfahren nach mindestens einem der Ansprüche 1 bis 5, **dadurch gekennzeichnet**, daß das DNA-Molekül durch reverse Transkription mit Reverser Transkriptase von einem gereinigten RNA-Molekül des westlichen Subtyps des FSME-Virus hergestellt wird.

7. Verfahren nach mindestens einem der Ansprüche 1 bis 5, **dadurch gekennzeichnet**, daß das DNA-Molekül durch chemische DNA-Synthese hergestellt wird.

8. Verfahren zur Herstellung eines DNA-Moleküls, das mit einem DNA-Molekül, hergestellt nach einem der Ansprüche 1 bis 7, unter stringenten Bedingungen hybridisiert, durch Isolieren der unter stringenten Bedingungen hybridisierenden DNA-Moleküle oder durch chemische Synthese.

9. Verfahren nach mindestens einem der Ansprüche 1 bis 8, **dadurch gekennzeichnet**, daß das DNA-Molekül DNA-Sequenzen umfaßt, die sich von den DNA-Molekülen, hergestellt nach den Ansprüchen 1 bis 8 als Folge von Degeneration des genetischen Codes und/oder Mutationen und/oder Transpositionen unterscheiden, aber noch für ein Polypeptid mit mindestens einer antigenen Determinante eines der Proteine kodieren, die aus der die Proteine C, prM, M oder E des westlichen Subtyps des FSME-Virus umfassenden Gruppe ausgewählt sind.

10. Verfahren nach mindestens einem der Ansprüche 1 bis 9, **dadurch gekennzeichnet**, daß das DNA-Molekül mit zusätzlichen DNA-Sequenzen kombiniert ist.

11. Verfahren nach Anspruch 10, **dadurch gekennzeichnet**, daß die zusätzlichen DNA-Sequenzen Replikation und Expression des DNA-Moleküls in einer Zellkultur erlauben und Sequenzen umfassen, die aus der Promotoren, Enhancer, Polyadenylierungssignale und Splicesignale umfassenden Gruppe ausgewählt sind.

12. Verfahren zur Herstellung eines RNA-Moleküls, das mindestens eine von der RNA des westlichen Subtyps des FSME-Virus abgeleitete RNA-Sequenz umfaßt und für ein oder mehrere Polypeptide mit den in Figur 1 dargestellten Aminosäuresequenzen kodiert, umfassend die Schritte
- Isolieren und Reinigen von RNA des festlichen Subtyps des FSME-Virus oder
- Herstellen des RNA-Moleküls durch rekombinante RNA/DNA-Techniken.

13. Verfahren nach Anspruch 12, **dadurch gekennzeichnet**, daß das RNA-Molekül die gesamte RNA-Sequenz umfaßt, die für die Strukturproteine kodiert.

14. Verfahren nach Anspruch 13, **dadurch gekennzeichnet**, daß das RNA-Molekül die in Figur 2 dargestellte Sequenz umfaßt.

15. Verfahren nach Anspruch 12, **dadurch gekennzeichnet**, daß das RNA-Molekül für eines der Proteine kodiert, die aus der die Proteine C, prM, M oder E des westlichen Subtyps des FSME-Virus umfassenden Gruppe ausgewählt sind.

16. Verfahren nach mindestens einem der Ansprüche 12 bis 15, **dadurch gekennzeichnet**, daß das RNA-Molekül für den Bereich mindestens einer antigenen Determinante eines der Proteine kodiert, die aus der die Proteine C, prM, M oder E des westlichen Subtyps des FSME-Virus umfassenden Gruppe ausgewählt sind.

17. Verfahren nach Anspruch 15, **dadurch gekennzeichnet**, daß das RNA-Molekül eine RNA-Sequenz umfaßt, die mindestens für eine antigene Determinante des Proteins E kodiert.

18. Verfahren nach mindestens einem der Ansprüche 12 bis 17, **dadurch gekennzeichnet**, daß das RNA-Molekül durch Isolieren und Reinigen von RNA des westlichen Subtyps des FSME-Virus oder durch rekombinante RNA/DNA-Techniken erhalten wird.

19. Verfahren nach Anspruch 18, **dadurch gekennzeichnet**, daß das RNA-Molekül durch Transkribieren isolierter und gereinigter Virus-RNA in DNA durch Reverse Transkriptase und anschließender Transkription dieser DNA wiederum in RNA erhalten wird.

20. Verfahren zur Herstellung eines RNA-Moleküls, dessen komplementärer Strang unter stringenten Bedingungen mit einem RNA-Molekül, hergestellt nach einem der Ansprüche 12 bis 18, hybridisiert, durch Isolieren des unter stringenten Bedingungen hybridisierenden RNA-Moleküls.

21. Verfahren zur Herstellung eines Vektors, der Insertionen von DNA-Sequenzen, hergestellt nach einem der Ansprüche 1 bis 11, oder RNA-Sequenzen, hergestellt nach einem der Ansprüche 12 bis 20, umfaßt, wobei der Vektor aus der Plasmide, Viren und Cosmide umfassenden Gruppe ausgewählt ist, durch Kombinieren der Vektorelemente nach Standardverfahren.

22. Verfahren nach Anspruch 21, **dadurch gekennzeichnet**, daß der Vektor das Plasmid pBR322 ist, das eine DNA-Sequenz enthält, die kodiert für ein Protein, ausgewählt aus Protein C, prM, M oder E des westlichen Subtyps des FSME-Virus oder Teilen davon.

23. Verfahren nach Anspruch 22, **dadurch gekennzeichnet**, daß der Vektor das bei der DSM mit der Hinterlegungsnummer DSM 4382 hinterlegte Plasmid A5, wie in Figur 5 dargestellt, ist.

24. Verfahren nach Anspruch 22, **dadurch gekennzeichnet**, daß der Vektor das bei der DSM mit der Hinterlegungsnummer DSM 4383 hinterlegte Plasmid P1-1 ist.

25. Verfahren nach mindestens einem der Ansprüche 21 oder 22, **dadurch gekennzeichnet**, daß der Vektor ein Insertionsplasmid ist, das über eine homologe Rekombination DNA-Sequenzen für das Protein E oder Teile davon in ein HindIII J-Fragment eines Vaccinia-Virus überträgt.

26. Verfahren nach Anspruch 25, **dadurch gekennzeichnet**, daß der Vektor das Plasmid pSC11-H1 gemäß Figur 7 ist, das die Nukleotide 6503-8051 der für Protein E kodierenden DNA-Sequenz enthält.

27. Verfahren nach Anspruch 25, **dadurch gekennzeichnet**, daß der Vektor das Plasmid pSC11-F41 gemäß Figur 7 ist, das die Nukleotide 6503-7998 der für Protein E kodierenden DNA-Sequenz enthält.

28. Verfahren nach Anspruch 25, **dadurch gekennzeichnet**, daß der Vektor das Plasmid pSC11-P6 gemäß Figur 7 ist, das die Nukleotide 6503-7524 der für Protein E kodierenden DNA-Sequenz enthält.

29. Verfahren zur Herstellung eines Vaccinia-Expressions-Vektors, der über homologe Rekombination DNA-Sequenzen für das Protein E enthält, bevorzugt inserierte DNA-Sequenzen aus einem der Plasmide gemäß einem der Ansprüche 25 bis 28, und zur Expression von Protein E oder Teilpeptiden von Protein E, enthaltend die zu den genannten DNA-Sequenzen korrespondierenden Aminosäuresequenzen, fähig ist, durch Inserieren der DNA-Sequenzen in die Vaccinia-Expressions-Vektoren mit Hilfe von Insertionsplasmiden.

30. Verfahren zur Herstellung eines Vehikels, das Insertionen von DNA-Sequenzen nach einem der Ansprüche 1 bis 11, oder RNA-Sequenzen nach einem der Ansprüche 12 bis 20 umfaßt, wobei das Vehikel ein Bakterium ist, durch Transformation des Vehikels mit den DNA- bzw. RNA-Sequenzen.

31. Verfahren nach Anspruch 30, **dadurch gekennzeichnet**, daß das Vehikel das Bakterium Salmonella typhi ist.

32. Verfahren zur Herstellung einer Zellkultur, die irgendeine der Sequenzen nach den Ansprüchen 1 bis 20 enthält, durch Transformieren von Zellen mit den Sequenzen.

33. Verfahren nach Anspruch 32, **dadurch gekennzeichnet**, daß die Zellkultur die Expression von Polypeptiden, die durch die RNA- oder DNA-Sequenzen nach einem der Ansprüche 1 bis 20 kodiert werden, erlaubt.

34. Verfahren nach Anspruch 33, **dadurch gekennzeichnet**, daß die Zellkultur eine Säugetier-Zellkultur ist.

35. Verfahren nach einem der Ansprüche 32 bis 34, **dadurch gekennzeichnet**, daß die Zellkultur eine Vero-Zellkultur oder eine Hühnerembryo-Fibroblastenzellkultur ist, die einen Vaccinia-Expressions-Vektor gemäß Anspruch 30 enthält, und in der der den inserierten DNA-Sequenzen entsprechende Teil des Proteins E exprimiert wird.

36. Verfahren zur Herstellung eines Peptids oder Polypeptids mit mindestens einer antigenen Determinante, wobei das Peptid oder Polypeptid eine Aminosäuresequenz umfaßt, die in der Aminosäuresequenz von Figur 3 enthalten ist, durch Expression einer Sequenz nach einem der Ansprüche 1 bis 20 oder durch chemische Synthese.

37. Verfahren nach Anspruch 36, **dadurch gekennzeichnet**, daß das Peptid oder Polypeptid durch Expression einer der Sequenzen nach einem der Ansprüche 1 bis 20 in einem geeigneten Expressionssystem hergestellt wird.

38. Verfahren nach Anspruch 37, **dadurch gekennzeichnet**, daß das Peptid oder Polypeptid in einer Zellkultur gemäß Anspruch 35 hergestellt wird.

39. Verfahren nach Anspruch 36, **dadurch gekennzeichnet**, daß das Peptid oder Polypeptid chemisch synthetisiert wird.

40. Zusammensetzung, **dadurch gekennzeichnet**, daß sie eines oder mehrere der Peptide oder Polypeptide nach einem der Ansprüche 36 bis 39 umfaßt.

41. Verfahren zur Herstellung eines Lebend-Impfstoffs, der DNA-Sequenzen nach einem der Ansprüche 1 bis 11 und/oder RNA-Sequenzen nach einem der Ansprüche 12 bis 20 umfaßt, wobei das Verfahren umfaßt Einführen der DNA- oder RNA-Sequenzen in die Genome lebender Viren.

42. Verfahren zur Herstellung eines Lebend-Impfstoffs, der eine oder mehrere der DNA-Sequenzen nach einem der Ansprüche 1 bis 11 mit Vaccinia-Virus DNA kombiniert enthält, wobei das Verfahren umfaßt Kombinieren der DNA-Sequenzen mit der DNA des Vaccinia-Virus.

43. Verfahren nach Anspruch 42, **dadurch gekennzeichnet**, daß der Lebend-Impfstoff einen Vaccinia-Vektor gemäß Anspruch 29 enthält.

44. Verfahren zur Herstellung eines Impfstoffs, der eine Zusammensetzung nach Anspruch 40 enthält, durch Kombinieren eines oder mehrerer der Peptide oder Polypeptide mit üblichen Hilfsstoffen.

45. Verwendung eines der Impfstoffe nach den Ansprüchen 41 bis 44 zum Herstellen spezifischer Immunglobuline.

46. Diagnostisches Mittel, **dadurch gekennzeichnet**, daß es eine Zusammensetzung nach Anspruch 40 enthält.

47. Verfahren zur Herstellung einer DNA- oder RNA-Sonde, die unter stringenten Bedingungen mit DNA- oder RNA-Sequenzen nach einem der Ansprüche 1 bis 9 und 12 bis 20 hybridisiert, durch rekombinante RNA/DNA-Technik oder durch chemische Synthese.

## Claims (Claims for the following Contracting State(s): AT, BE, CH, LI, DE, FR, GB, GR, IT, LU, NL, SE)

1. DNA molecule, characterized in that it encompasses at least one DNA sequence derived from the western subtype of the TBE (tick-borne encephalitis) virus, which DNA sequence encodes one or more polypeptides from the group comprising the proteins C, prM, M or E of the western subtype of the TBE virus, where the polypeptide encompasses an amino acid sequence which is contained in the amino acid sequence depicted in Figure 3.

2. DNA molecule according to Claim 1, characterized in that the polypeptide encompasses the region of one or more antigenic determinants of one of the proteins from the group comprising the proteins C, prM, M or E of the western subtype of the TBE virus.

3. DNA molecule according to Claim 1, characterized in that it encompasses the entire DNA sequence encoding the structural proteins of the TBE virus.

4. DNA molecule according to Claim 3, characterized in that it encompasses the sequence depicted in Figure 1.

5. DNA molecule according to Claim 2, characterized in that it encompasses the DNA sequence encoding the region of one antigenic determinant of the protein E.

6. DNA molecule according to at least one of Claims 1 to 5, characterized in that it was obtained from a purified RNA molecule of the western subtype of the TBE virus by reverse transcription using reverse transcriptase.

7. DNA molecule according to at least one of Claims 1 to 5, characterized in that it is prepared by chemical DNA synthesis.

8. DNA molecule, characterized in that it hybridises under stringent conditions with a DNA molecule according to one of Claims 1 to 7.

9. DNA molecule according to at least one of Claims 1 to 8, characterized in that it encompasses DNA sequences which differ from the DNA molecules according to Claims 1 to 8 as a consequence of degeneration of the genetic code and/or mutations and/or transpositions, but which still encode a polypeptide containing at least one antigenic determinant of one of the proteins which are selected from the group comprising the proteins C, prM, M or E of the western subtype of the TBE virus.

10. DNA molecule according to at least one of Claims 1 to 9, characterized in that it is combined with additional DNA sequences.

11. DNA molecule according to Claim 10, characterized in that the additional DNA sequences permit replication and expression of the DNA molecule in a cell culture and encompass sequences which are selected from the group comprising promoters, enhancers, polyadenylation signals and splice signals.

12. RNA molecule, characterized in that it encompasses at least one RNA sequence derived from the RNA of the western subtype of the TBE virus and encodes one or more polypeptides having the amino acid sequences depicted in Figure 3.

13. RNA molecule according to Claim 12, characterized in that it encompasses the entire RNA sequence which encodes the structural proteins.

14. RNA molecule according to Claim 13, characterized in that it encompasses the sequence depicted in Figure 2.

15. RNA molecule according to Claim 12, characterized in that it encodes one of the proteins which are selected from the group comprising the proteins C, prM, M or E of the western subtype of the TBE virus.

16. RNA molecule according to at least one of Claims 12 to 15, characterized in that it encodes the region of at least one antigenic determinant of one of the proteins which are selected from the group comprising the proteins C, prM, M or E of the western subtype of the TBE virus.

17. RNA molecule according to Claim 15, characterized in that it encompasses RNA sequences encoding the region of at least one antigenic determinant of the protein E.

18. RNA molecule according to at least one of Claims 12 to 17, characterized in that it is obtained by isolating and purifying RNA of the western subtype of the TBE virus or by recombinant RNA/DNA techniques.

19. RNA molecule according to Claim 18, characterized in that it is obtained by transcribing isolated and purified viral RNA into DNA using reverse transcriptase and subsequently transcribing this DNA once again into RNA.

20. RNA molecule, characterized in that its complementary strand hybridises under stringent conditions with an RNA molecule according to one of Claims 12 to 18.

21. Vector, characterized in that it encompasses insertions of DNA sequences according to one of Claims 1 to 11 or RNA sequences according to one of Claims 12 to 20, where the vector is selected from the group comprising plasmids, viruses and cosmids.

22. Vector according to Claim 21, characterized in that it is the plasmid pBR322 which contains a DNA sequence which encodes a protein selected from the group comprising the proteins C, prM, M or E of the western subtype of the TBE virus, or parts thereof.

23. Vector according to Claim 22, characterized in that it is the plasmid A5, as depicted in Figure 5, which is deposited at the DSM (German Collection of Microorganisms) under the deposition number DSM 4382.

24. Vector according to Claim 22, characterized in that it is the plasmid P1-1 which is deposited at the DSM under the deposition number DSM 4383.

25. Vector according to at least one of Claims 21 or 22, characterized in that it is an insertion plasmid which transfers DNA sequences for the protein E, or parts thereof, by way of homologous recombination, into a HindIII J fragment of a vaccinia virus.

26. Vector according to Claim 25, characterized in that it is the plasmid pSC11-H1 according to Figure 7, which plasmid contains the nucleotides 6503-8051 of the DNA sequence encoding protein E.

27. Vector according to Claim 25, characterized in that it is the plasmid pSC11-F41 according to Figure 7, which plasmid contains the nucleotides 6503-7998 of the DNA sequence encoding protein E.

28. Vector according to Claim 25, characterized in that it is the plasmid pSC11-P6 according to Figure 7, which plasmid contains the nucleotides 6503-7524 of the DNA sequence encoding protein E.

29. Vaccinia expression vector, characterized in that it contains, by way of homologous recombination, DNA sequences for the protein E, preferably inserted DNA sequences from one of the plasmids according to one of Claims 25-28, and is capable of expressing protein E or constituent peptides of protein E containing the amino acid sequences corresponding to the said DNA sequences.

30. Vehicle, characterized in that it encompasses insertions of DNA sequences according to one of Claims 1-11 or RNA sequences according to one of Claims 12-20, where the vehicle is a bacterium.

31. Vehicle according to Claim 30, characterized in that the bacterium is Salmonella typhi.

32. Cell culture, characterized in that it contains any one of the sequences according to Claims 1 to 20.

33. Cell culture according to Claim 32, characterized in that it permits the expression of polypeptides which are encoded by the RNA or DNA sequences according to one of Claims 1 to 20.

34. Cell culture according to Claim 33, characterized in that the cell culture is a mammalian cell culture.

35. Cell culture according to one of Claims 32 to 34, characterized in that it is a Vero cell culture or a chick-embryo fibroblast cell culture which contains a vaccinia expression vector according to Claim 30 and in which the part of the protein E corresponding to the inserted DNA sequences is expressed.

36. Peptide or polypeptide containing at least one antigenic determinant, where the peptide or polypeptide encompasses an amino acid sequence which is contained in the amino acid sequence of Figure 3.

37. Peptide or polypeptide according to Claim 36, characterized in that it is prepared by expressing one of the sequences according to one of Claims 1 to 20 in a suitable expression system.

38. Peptide or polypeptide according to Claim 37, characterized in that it is prepared in a cell culture according to Claim 35.

39. Peptide or polypeptide according to Claim 36, characterized in that it is synthesized chemically.

40. Composition, characterized in that it comprises one or more of the peptides or polypeptides according to one of Claims 36 to 39.

41. Living vaccine, characterized in that it comprises DNA sequences according to one of Claims 1 to 11 and/or RNA sequences according to one of Claims 12 to 20.

42. Living vaccine, characterized in that one or more of the DNA sequences according to one of Claims 1 to 11 are combined with vaccinia virus DNA.

43. Living vaccine according to Claim 42, characterized in that it contains a vaccinia vector according to Claim 29.

44. Vaccine, characterized in that it contains a composition according to Claim 40.

45. Use of one of the vaccines according to Claims 41 to 44 for preparing specific immunoglobulins.

46. Diagnostic agent, characterized in that it contains a composition according to Claim 40.

47. DNA or RNA probe, characterized in that it hybridises under stringent conditions with DNA or RNA sequences according to one of Claims 1 to 9 and 12 to 20.

## Claims (Claims for the following Contracting State(s): ES)

1. Process for preparing a DNA molecule which encompasses at least one DNA sequence derived from the western subtype of the TBE (tick-borne encephalitis) virus, which sequence encodes one or more polypeptides from the group comprising the proteins C, prM, M or E of the western subtype of the TBE virus, where the polypeptide encompasses an amino acid sequence which is contained in the amino acid sequence depicted in Figure 3, comprising the steps
- purification of the RNA molecule, and
- transcription of the RNA template into the DNA molecule using reverse transcriptase; or
- chemical synthesis of the DNA molecule.

2. Process according to Claim 1, characterized in that the polypeptide encompasses the region of one or more antigenic determinants of one of the proteins from the group comprising the proteins C, prM, M or E of the western subtype of the TBE virus.

3. Process according to Claim 1, characterized in that the DNA molecule encompasses the entire DNA sequence encoding the structural proteins of the TBE virus.

4. Process according to Claim 3, characterized in that the DNA molecule encompasses the sequence depicted in Figure 1.

5. Process according to Claim 2, characterized in that the DNA molecule encompasses the DNA sequence encoding the region of an antigenic determinant of the protein E.

6. Process according to at least one of Claims 1 to 5, characterized in that the DNA molecule is prepared from a purified RNA molecule of the western subtype of the TBE virus by means of reverse transcription using reverse transcriptase.

7. Process according to at least one of Claims 1 to 5, characterized in that the DNA molecule is prepared by chemical DNA synthesis.

8. Process for preparing a DNA molecule, which hybridises under stringent conditions with a DNA molecule prepared according to one of Claims 1 to 7, by isolating the DNA molecules which hybridise under stringent conditions or by chemical synthesis.

9. Process according to at least one of Claims 1 to 8, characterized in that the DNA molecule encompasses DNA sequences which differ from the DNA molecules prepared according to Claims 1 to 8 as a consequence of degeneration of the genetic code and/or mutations and/or transpositions, but which still encode a polypeptide containing at least one antigenic determinant of one of the proteins which are selected from the group comprising the proteins C, prM, M or E of the western subtype of the TBE virus.

10. Process according to at least one of Claims 1 to 9, characterized in that the DNA molecule is combined with additional DNA sequences.

11. Process according to Claim 10, characterized in that the additional DNA sequences permit replication and expression of the DNA molecule in a cell culture, and encompass sequences which are selected from the group comprising promoters, enhancers, polyadenylation signals and splice signals.

12. Process for preparing an RNA molecule which encompasses at least one RNA sequence derived from the RNA of the western subtype of the TBE virus and which encodes one or more polypeptides containing the amino acid sequences depicted in Figure 1, comprising the steps
- isolation and purification of RNA of the western subtype of the TBE virus, or
- preparation of the RNA molecule by recombinant RNA/DNA techniques.

13. Process according to Claim 12, characterized in that the RNA molecule encompasses the entire RNA sequence which encodes the structural proteins.

14. Process according to Claim 13, characterized in that the RNA molecule encompasses the sequence depicted in Figure 2.

15. Process according to Claim 12, characterized in that the RNA molecule encodes one of the proteins which are selected from the group comprising the proteins C, prM, M or E of the western subtype of the TBE virus.

16. Process according to at least one of Claims 12 to 15, characterized in that the RNA molecule encodes the region of at least one antigenic determinant of one of the proteins which are selected from the group comprising the proteins C, prM, M or E of the western subtype of the TBE virus.

17. Process according to Claim 15, characterized in that the RNA molecule encompasses an RNA sequence which encodes at least one antigenic determinant of the protein E.

18. Process according to at least one of Claims 12 to 17, characterized in that the RNA molecule is obtained by isolating and purifying RNA of the western subtype of the TBE virus or by recombinant RNA/DNA techniques.

19. Process according to Claim 18, characterized in that the RNA molecule is obtained by transcribing isolated and purified viral RNA into DNA using reverse transcriptase and subsequently transcribing this DNA once again into RNA.

20. Process for preparing an RNA molecule, whose complementary strand hybridises under stringent conditions with an RNA molecule prepared according to one of Claims 12 to 18, by isolating the RNA molecule which hybridises under stringent conditions.

21. Process for preparing a vector which encompasses insertions of DNA sequences prepared according to one of Claims 1 to 11 or RNA sequences prepared according to one of Claims 12 to 20, where the vector is selected from the group comprising plasmids, viruses and cosmids, by combining the vector elements in accordance with standard methods.

22. Process according to Claim 21, characterized in that the vector is the plasmid pBR322 which contains a DNA sequence which encodes a protein selected from protein C, prM, M or E of the western subtype of the TBE virus, or parts thereof.

23. Process according to Claim 22, characterized in that the vector is the plasmid A5, as depicted in Figure 5, which is deposited at the DSM (German collection of microorganisms) under the deposition number DSM 4382.

24. Process according to Claim 22, characterized in that the vector is the plasmid P1-1 which is deposited at the DSM under the deposition number DSM 4383.

25. Process according to at least one of Claims 21 or 22, characterized in that the vector is an insertion plasmid which transfers DNA sequences for the protein E, or parts thereof, by way of homologous recombination, into a HindIII J fragment of a vaccinia virus.

26. Process according to Claim 25, characterized in that the vector is the plasmid pSC11-H1 in accordance with Figure 7, which plasmid contains the nucleotides 6503-8051 of the DNA sequence encoding protein E.

27. Process according to Claim 25, characterized in that the vector is the plasmid pSC11-F41 in accordance with Figure 7, which plasmid contains the nucleotides 6503-7998 of the DNA sequence encoding protein E.

28. Process according to Claim 25, characterized in that the vector is the plasmid pSC11-P6 in accordance with Figure 7, which plasmid contains the nucleotides 6503-7524 of the DNA sequence encoding protein E.

29. Process for preparing a vaccinia expression vector which contains, by way of homologous recombination, DNA sequences for the protein E, preferably inserted DNA sequences from one of the plasmids according to one of Claims 25 to 28, and which is capable of expressing protein E or constituent peptides of protein E containing the amino acid sequences corresponding to the said DNA sequences, by means of inserting the DNA sequences into the vaccinia expression vectors using insertion plasmids.

30. Process for preparing a vehicle which encompasses insertions of DNA sequences according to one of Claims 1 to 11 or RNA sequences according to one of Claims 12 to 20, where the vehicle is a bacterium, by means of transforming the vehicle with the DNA or RNA sequences, respectively.

31. Process according to Claim 30, characterized in that the vehicle is the bacterium Salmonella typhi.

32. Process for preparing a cell culture, which contains any one of the sequences according to Claims 1 to 20, by means of transforming cells with the sequences.

33. Process according to Claim 32, characterized in that the cell culture permits the expression of polypeptides which are encoded by the RNA or DNA sequences according to one of Claims 1 to 20.

34. Process according to Claim 33, characterized in that the cell culture is a mammalian cell culture.

35. Process according to one of Claims 32 to 34, characterized in that the cell culture is a Vero cell culture or a chick-embryo fibroblast cell culture which contains a vaccinia expression vector according to Claim 30 and in which the part of the protein E corresponding to the inserted DNA sequences is expressed.

36. Process for preparing a peptide or polypeptide containing at least one antigenic determinant, where the peptide or polypeptide encompasses an amino acid sequence which is contained in the amino acid sequence of Figure 3, by means of expressing a sequence according to one of Claims 1 to 20 or by means of chemical synthesis.

37. Process according to Claim 36, characterized in that the peptide or polypeptide is prepared by means of expressing one of the sequences according to one of Claims 1 to 20 in a suitable expression system.

38. Process according to Claim 37, characterized in that the peptide or polypeptide is prepared in a cell culture according to Claim 35.

39. Process according to Claim 36, characterized in that the peptide or polypeptide is synthesized chemically.

40. Composition, characterized in that it comprises one or more of the peptides or polypeptides according to one of Claims 36 to 39.

41. Process for preparing a living vaccine which comprises DNA sequences according to one of Claims 1 to 11 and/or RNA sequences according to one of Claims 12 to 20, where the process comprises introducing the DNA or RNA sequences into the genomes of living viruses.

42. Process for preparing a living vaccine which contains one or more of the DNA sequences according to one of Claims 1 to 11 in combination with vaccinia virus DNA, where the process comprises combining the DNA sequences with the DNA of the vaccinia virus.

43. Process according to Claim 42, characterized in that the living vaccine contains a vaccinia vector according to Claim 29.

44. Process for preparing a vaccine which contains a composition according to Claim 40, by means of combining one or more of the peptides or polypeptides with customary auxiliary substances.

45. Use of one of the vaccines according to Claims 41 to 44 for preparing specific immunoglobulins.

46. Diagnostic agent, characterized in that it contains a composition according to Claim 40.

47. Process for preparing a DNA or RNA probe, which hybridises under stringent conditions with DNA or RNA sequences according to one of Claims 1 to 9 and 12 to 20, by means of a recombinant RNA/DNA technique or by means of chemical synthesis.

## Revendications (Revendications pour l'(les) Etat(s) contractant(s) suivant(s): AT, BE, CH, LI, DE, FR, GB, GR, IT, LU, NL, SE)

1. Molécule d'ADN, caractérisée en ce qu'elle comprend au moins une séquence d'ADN dérivée du virus MEVE sous-type de l'ouest qui code pour un ou plusieurs polypeptides du groupe des protéines C, prM, M ou E du virus MEVE sous-type de l'ouest, le polypeptide comportant une séquence d'amino-acides qui est contenue dans la séquence d'amino-acides représentée sur la figure 3.

2. Molécule d'ADN selon la revendication 1, caractérisée en ce que le polypeptide comprend le domaine d'un ou plusieurs déterminants antigéniques de l'une des protéines du groupe des protéines C, prM, M ou E du virus MEVE sous-type de l'ouest.

3. Molécule d'ADN selon la revendication 1, caractérisée en ce qu'elle comprend l'ensemble de la séquence d'ADN codant pour la protéine de structure du virus MEVE.

4. Molécule d'ADN selon la revendication 3, caractérisée en ce qu'elle comprend la séquence représentée sur la figure 1.

5. Molécule d'ADN selon la revendication 2, caractérisée en ce qu'elle comprend la séquence d'ADN codant pour le domaine d'un déterminant antigénique de la protéine E.

6. Molécule d'ADN selon l'une au moins des revendications 1 à 5, caractérisée en ce qu'elle a été obtenue par transcription inverse avec une transcriptase inverse à partir d'une molécule d'ARN purifiée du virus MEVE sous-type de l'ouest.

7. Molécule d'ADN selon l'une au moins des revendications 1 à 5, caractérisée en ce qu'elle est produite par synthèse chimique d'ADN.

8. Molécule d'ADN, caractérisée en ce qu'elle hybride dans des conditions stringentes avec une molécule d'ADN selon l'une des revendications 1 à 7.

9. Molécule d'ADN selon l'une au moins des revendications 1 à 8, caractérisée en ce qu'elle comprend des séquences d'ADN qui se distinguent des molécules d'ADN selon l'une des revendications 1 à 8 par suite de la dégénérescence du code génétique et/ou de mutations et/ou de transpositions, mais qui codent encore pour un polypeptide comportant au moins un déterminant antigénique de l'une des protéines qui sont choisies dans le groupe des protéines C, prM, M ou E du virus MEVE sous-type de l'ouest.

10. Molécule d'ADN selon l'une au moins des revendications 1 à 9, caractérisée en qu'elle est combinée avec des séquences d'ADN supplémentaires.

11. Molécule d'ADN selon la revendication 10, caractérisée en ce que les séquences d'ADN supplémentaires permettent la réplication et l'expression de la molécule d'ADN dans une culture cellulaire et comprennent des séquences qui sont choisies dans le groupe comprenant les promoteurs, les "Enhancer", les signaux de polyadénylation et les signaux d'épissage.

12. Molécule d'ARN, caractérisée en ce qu'elle comprend au moins une séquence d'ARN dérivée de l'ARN du virus MEVE sous-type de l'ouest, et qu'elle code pour un ou plusieurs polypeptides avec les séquences d'amino-acides représentées à la figure 3.

13. Molécule d'ARN selon la revendication 12, caractérisée en ce qu'elle comprend l'ensemble de la séquence d'ARN qui code pour la protéine de structure.

14. Molécule d'ARN selon la revendication 13, caractérisée en ce qu'elle comprend la séquence représentée sur la figure 2.

15. Molécule d'ARN selon la revendication 12, caractérisée en ce qu'elle code pour l'une des protéines qui sont choisies dans le groupe des protéines C, prM, M ou E du virus MEVE sous-type de l'ouest.

16. Molécule d'ARN selon l'une au moins des revendications 12 à 15, caractérisée en ce qu'elle code pour le domaine d'au moins un déterminant antigénique de l'une des protéines qui sont choisies dans le groupe des protéines C, prM, M ou E du virus MEVE sous-type de l'ouest.

17. Molécule d'ARN selon la revendication 15, caractérisée en ce qu'elle comprend des séquences d'ARN codant pour le domaine d'au moins un déterminant antigénique de la protéine E.

18. Molécule d'ARN selon l'une au moins des revendications 12 à 17, caractérisée en ce qu'elle est obtenue par isolement et purification d'ARN du virus MEVE sous-type de l'ouest ou par des techniques de l'ARN/ADN recombinant.

19. Molécule d'ARN selon la revendication 18, caractérisée en ce qu'elle est obtenue par transcription d'ARN viral isolé et purifié en ADN à l'aide d'une transcriptase inverse, et en transcrivant ensuite cet ADN de nouveau en ARN.

20. Molécule d'ARN, caractérisée en ce que son caténaire complémentaire hybride dans des conditions stringentes avec une molécule d'ARN selon l'une des revendications 12 à 18.

21. Vecteur, caractérisé en ce qu'il comprend des insertions de séquences d'ADN selon l'une des revendications 1 à 11 ou de séquences d'ARN selon l'une des revendications 12 à 20, le vecteur étant choisi dans le groupe comprenant les plasmides, les virus et les cosmides.

22. Vecteur selon la revendication 21, caractérisé en ce qu'il est le plasmide pBR322, qui contient une séquence d'ADN qui code pour une protéine choisie parmi les protéines C, prM, M ou E du virus MEVE sous-type de l'ouest ou de parties de ce virus.

23. Vecteur selon la revendication 22, caractérisé en ce qu'il est le plasmide A5 déposé auprès de la DSM sous le numéro de dépôt DSM 4382, comme représenté à la figure 5.

24. Vecteur selon la revendication 22, caractérisé en ce qu'il est le plasmide P1-1 déposé auprès de la DSM sous le numéro de dépôt DSM 4383.

25. Vecteur selon l'une au moins des revendications 21 ou 22, caractérisé en ce qu'il est un plasmide d'insertion qui, par une recombinaison homologue, transfère des séquences d'ADN pour la protéine E ou des parties de cette protéine dans un fragment J-HindIII d'un virus de la vaccine.

26. Vecteur selon la revendication 25, caractérisé en qu'il est le plasmide pSC11-H1 selon la figure 7 qui contient les nucléotides 6503-8051 de la séquence d'ADN codant pour la protéine E.

27. Vecteur selon la revendication 25, caractérisé en ce qu'il est le plasmide pSC11-F41 selon la figure 7 qui contient les nucléotides 6503-7998 de la séquence d'ADN codant pour la protéine E.

28. Vecteur selon la revendication 25, caractérisé en ce qu'il est le plasmide pSC11-P6 selon la figure 7 qui contient les nucléotides 6503-7524 de la séquence d'ADN codant pour la protéine E.

29. Vecteur d'expression de la vaccine, caractérisé en ce qu'il contient par recombinaison homologue des séquences d'ADN pour la protéine E, de préférence des séquences d'ADN insérées à partir de l'un des plasmides selon l'une des revendications 25 à 28, et en ce qu'il est en mesure d'exprimer la protéine E ou des peptides partiels de la protéine E contenant les séquences d'amino-acides correspondant aux séquences d'ADN mentionnées.

30. Véhicule, caractérisé en ce qu'il comprend des insertions de séquences d'ADN selon l'une des revendications 1 à 11 ou de séquences d'ARN selon l'une des revendications 12 à 20, le véhicule étant une bactérie.

31. Véhicule selon la revendication 30, caractérisé en ce que la bactérie est Salmonella typhi.

32. Culture cellulaire, caractérisée en ce qu'elle contient l'une quelconque des séquences selon les revendications 1 à 20.

33. Culture cellulaire selon la revendication 32, caractérisée en ce qu'elle permet l'expression de polypeptides qui sont codés par les séquences d'ARN ou d'ADN selon l'une des revendications 1 à 20.

34. Culture cellulaire selon la revendication 33, caractérisée en ce que la culture cellulaire est une culture cellulaire de mammifère.

35. Culture cellulaire selon l'une des revendications 32 à 34, caractérisée en ce qu'il s'agit d'une culture de cellules Vero ou d'une culture cellulaire de fibroblastes d'embryon de poulet qui contient un vecteur d'expression de la vaccine selon la revendication 30, et dans laquelle est exprimée la partie de la protéine E correspondant aux séquences d'ADN insérées.

36. Peptide ou polypeptide comportant au moins un déterminant antigénique, le peptide ou le polypeptide comprenant une séquence d'amino-acides qui est contenue dans la séquence d'amino-acides de la figure 3.

37. Peptide ou polypeptide selon la revendication 36, caractérisé en ce qu'il est produit dans un système d'expression approprié, par expression de l'une des séquences selon l'une des revendications 1 à 20.

38. Peptide ou polypeptide selon la revendication 37, caractérisé en ce qu'il est produit dans une culture cellulaire selon la revendication 35.

39. Peptide ou polypeptide selon la revendication 36, caractérisé en ce qu'il est obtenu par synthèse chimique.

40. Composition, caractérisée en ce qu'elle comprend un ou plusieurs peptides ou polypeptides selon l'une des revendications 36 à 39.

41. Vaccin vivant, caractérisé en ce qu'il comprend des séquences d'ADN selon l'une des revendications 1 à 11 et/ou des séquences d'ARN selon l'une des revendications 12 à 20.

42. Vaccin vivant, caractérisé en ce que l'une ou plusieurs des séquences d'ADN selon l'une des revendications 1 à 11 sont combinées avec l'ADN viral de la vaccine.

43. Vaccin vivant selon la revendication 42, caractérisé en ce qu'il contient un vecteur de la vaccine selon la revendication 29.

44. Vaccin, caractérisé en ce qu'il contient une composition selon la revendication 40.

45. Utilisation de l'un des vaccins selon les revendications 41 à 44 pour la production d'immunoglobulines spécifiques.

46. Agent diagnostique, caractérisé en ce qu'il contient une composition selon la revendication 40.

47. Sonde d'ADN ou d'ARN, caractérisée en ce qu'elle hybride dans des conditions stringentes avec des séquences d'ADN ou d'ARN selon l'une des revendications 1 à 9 et 12 à 20.

## Revendications (Revendications pour l'(les) Etat(s) contractant(s) suivant(s): ES)

1. Procédé pour la fabrication d'une molécule d'ADN comprenant au moins une séquence d'ADN dérivée du virus MEVE sous-type de l'ouest qui code pour un ou plusieurs polypeptides du groupe des protéines C, prM, M ou E du virus MEVE sous-type de l'ouest, le polypeptide comportant une séquence d'amino-acides qui est contenue dans la séquence d'amino-acides représentée sur la figure 3, englobant les phases de
- purification de la molécule d'ARN, et de
- transcription de la matrice d'ARN dans la molécule d'ADN à l'aide d'une transcriptase inverse ; ou de
- synthèse chimique de la molécule d'ADN.

2. Procédé selon la revendication 1, caractérisé en ce que polypeptide comprend le domaine d'un ou plusieurs déterminants antigéniques de l'une des protéines du groupe des protéines C, prM, M ou E du virus MEVE sous-type de l'ouest.

3. Procédé selon la revendication 1, caractérisé en ce que la molécule d'ADN comprend l'ensemble de la séquence d'ADN codant pour la protéine de structure du virus MEVE.

4. Procédé selon la revendication 3, caractérisé en ce que la molécule d'ADN comprend la séquence représentée sur la figure 1.

5. Procédé selon la revendication 2, caractérisé en ce que la molécule d'ADN comprend la séquence d'ADN codant pour le domaine d'un déterminant antigénique de la protéine E.

6. Procédé selon l'une au moins des revendications 1 à 5, caractérisé en ce que la molécule d'ADN est produite par transcription inverse avec une transcriptase inverse à partir d'une molécule d'ARN purifiée du virus MEVE sous-type de l'ouest.

7. Procédé selon l'une au moins des revendications 1 à 5, caractérisé en ce la molécule d'ADN est produite par synthèse chimique d'ADN.

8. Procédé pour la fabrication d'une molécule d'ADN qui hybride dans des conditions stringentes avec une molécule d'ADN produite selon l'une des revendications 1 à 7, par isolement des molécules d'ADN qui hybrident dans des conditions stringentes ou par synthèse chimique.

9. Procédé selon l'une au moins des revendications 1 à 8, caractérisé en ce que la molécule d'ADN comprend des séquences d'ADN qui se distinguent des molécules d'ADN produites selon l'une des revendications 1 à 8, par suite de la dégénérescence du code génétique et/ou de mutations et/ou de transpositions, mais qui codent encore pour un polypeptide comportant au moins un déterminant antigénique de l'une des protéines qui sont choisies dans le groupe des protéines C, prM, M ou E du virus MEVE sous-type de l'ouest.

10. Procédé selon l'une au moins des revendications 1 à 9, caractérisé en que la molécule d'ADN est combinée avec des séquences d'ADN supplémentaires.

11. Procédé selon la revendication 10, caractérisé en ce que les séquences d'ADN supplémentaires permettent la réplication et l'expression de la molécule d'ADN dans une culture cellulaire et comprennent des séquences qui sont choisies dans le groupe comprenant les promoteurs, les "Enhancer", les signaux de polyadénylation et les signaux d'épissage.

12. Procédé pour la fabrication d'une molécule d'ARN, qui comprend au moins une séquence d'ARN dérivée de l'ARN du virus MEVE sous-type de l'ouest, et qui code pour un ou plusieurs polypeptides avec les séquences d'amino-acides représentées à la figure 1, englobant les phases
- d'isolement et de purification d'ARN du virus MEVE sous-type de l'ouest, ou de
- production de la molécule d'ARN par des techniques de d'ARN/ADN recombinant.

13. Procédé selon la revendication 12, caractérisé en ce que la molécule d'ARN comprend l'ensemble de la séquence d'ARN qui code pour la protéine de structure.

14. Procédé selon la revendication 13, caractérisé en ce que la molécule d'ARN comprend la séquence représentée sur la figure 2.

15. Procédé selon la revendication 12, caractérisé en ce que la molécule d'ARN code pour l'une des protéines qui sont choisies dans le groupe des protéines C, prM, M ou E du virus MEVE sous-type de l'ouest.

16. Procédé selon l'une au moins des revendications 12 à 15, caractérisé en ce la molécule d'ARN code pour le domaine d'au moins un déterminant antigénique de l'une des protéines qui sont choisies dans le groupe des protéines C, prM, M ou E du virus MEVE sous-type de l'ouest.

17. Procédé selon la revendication 15, caractérisé en ce que la molécule d'ARN comprend une séquence d'ARN qui code pour le domaine d'au moins un déterminant antigénique de la protéine E.

18. Procédé selon l'une au moins des revendications 12 à 17, caractérisé en ce que la molécule d'ARN est obtenue par isolement et purification d'ARN du virus MEVE sous-type de l'ouest ou par des techniques de l'ARN/ADN recombinant.

19. Procédé selon la revendication 18, caractérisé en ce que la molécule d'ARN est obtenue par transcription d'ARN viral isolé et purifié en ADN à l'aide d'une transcriptase inverse, et en transcrivant ensuite cet ADN à nouveau en ARN.

20. Procédé pour la fabrication d'une molécule d'ARN dont le caténaire complémentaire hybride dans des conditions stringentes avec une molécule d'ARN produite selon l'une des revendications 12 à 18, par isolement de la molécule d'ARN qui hybride dans des conditions stringentes.

21. Procédé pour la fabrication d'un vecteur qui comprend des insertions de séquences d'ADN produites selon l'une des revendications 1 à 11, ou de séquences d'ARN produites selon l'une des revendications 12 à 20, le vecteur étant choisi dans le groupe comprenant les plasmides, les virus et les cosmides, par combinaison des éléments vecteurs selon des procédés standards.

22. Procédé selon la revendication 21, caractérisé en ce que le vecteur est le plasmide pBR322, qui contient une séquence d'ADN qui code pour une protéine choisie parmi les protéines C, prM, M ou E du virus MEVE sous-type de l'ouest ou de parties de ce virus.

23. Procédé selon la revendication 22, caractérisé en ce que le vecteur est le plasmide A5 déposé auprès de la DSM sous le numéro de dépôt DSM 4382, comme représenté sur la figure 5.

24. Procédé selon la revendication 22, caractérisé en ce que le vecteur est le plasmide P1-1 déposé auprès de la DSM sous le numéro de dépôt DSM 4383.

25. Procédé selon l'une au moins des revendications 21 ou 22, caractérisé en ce que le vecteur est un plasmide d'insertion qui, par une recombinaison homologue, transfère des séquences d'ADN pour la protéine E ou des parties de cette protéine dans un fragment J-HindIII d'un virus de la vaccine.

26. Procédé selon la revendication 25, caractérisé en que le vecteur est le plasmide pSC11-H1 selon la figure 7 qui contient les nucléotides 6503-8051 de la séquence d'ADN codant pour la protéine E.

27. Procédé selon la revendication 25, caractérisé en ce que le vecteur est le plasmide pSC11-F41 selon la figure 7 qui contient les nucléotides 6503-7998 de la séquence d'ADN codant pour la protéine E.

28. Procédé selon la revendication 25, caractérisé en ce que le vecteur est le plasmide pSC11-P6 selon la figure 7 qui contient les nucléotides 6503-7524 de la séquence d'ADN codant pour la protéine E.

29. Procédé pour la fabrication d'un vecteur d'expression de la vaccine qui contient par recombinaison homologue des séquences d'ADN pour la protéine E, de préférence des séquences d'ADN insérées à partir de l'un des plasmides selon l'une des revendications 25 à 28, et en ce que, par insertion des séquences d'ADN dans les vecteurs d'expression de la vaccine à l'aide de plasmides d'insertion, il est en mesure d'exprimer la protéine E ou des peptides partiels de la protéine E contenant les séquences d'amino-acides correspondant aux séquences d'ADN mentionnées.

30. Procédé pour la fabrication d'un véhicule qui comprend des insertions de séquences d'ADN selon l'une des revendications 1 à 11 ou de séquences d'ARN selon l'une des revendications 12 à 20, le véhicule étant une bactérie, par transformation du véhicule avec les séquences d'ADN ou d'ARN.

31. Procédé selon la revendication 30, caractérisé en ce que la bactérie est Salmonella typhi.

32. Procédé pour la fabrication d'une culture cellulaire contenant l'une quelconque des séquences selon les revendications 1 à 20, par transformation de cellules avec les séquences.

33. Procédé selon la revendication 32, caractérisé en ce que la culture cellulaire permet l'expression de polypeptides qui sont codés par les séquences d'ARN ou d'ADN selon l'une des revendications 1 à 20.

34. Procédé selon la revendication 33, caractérisé en ce que la culture cellulaire est une culture cellulaire de mammifère.

35. Procédé selon l'une des revendications 32 à 34, caractérisé en ce que la culture cellulaire est une culture de cellules Vero ou une culture cellulaire de fibroblastes d'embryon de poulet qui contient un vecteur d'expression de la vaccine selon la revendication 30, et dans laquelle est exprimée la partie de la protéine E correspondant aux séquences d'ADN insérées.

36. Procédé pour la fabrication d'un peptide ou d'un polypeptide comportant au moins un déterminant antigénique, le peptide ou le polypeptide comprenant une séquence d'amino-acides qui est contenue dans la séquence d'amino-acides de la figure 3, par expression d'une séquence selon l'une des revendications 1 à 20 ou par synthèse chimique.

37. Procédé selon la revendication 36, caractérisé en ce que le peptide ou le polypeptide est produit dans un système d'expression approprié, par expression de l'une des séquences selon l'une des revendications 1 à 20.

38. Procédé selon la revendication 37, caractérisé en ce que le peptide ou le polypeptide est produit dans une culture cellulaire selon la revendication 35.

39. Procédé selon la revendication 36, caractérisé en ce que le peptide ou le polypeptide est obtenu par synthèse chimique.

40. Composition, caractérisée en ce qu'elle comprend un ou plusieurs peptides ou polypeptides selon l'une des revendications 36 à 39.

41. Procédé pour la fabrication d'un vaccin vivant qui comprend des séquences d'ADN selon l'une des revendications 1 à 11 et/ou des séquences d'ARN selon l'une des revendications 12 à 20, le procédé comprenant l'implantation des séquences d'ADN ou d'ARN dans les génomes de virus vivants.

42. Procédé pour la fabrication d'un vaccin vivant qui contient une ou plusieurs des séquences d'ADN selon l'une des revendications 1 à 11 qui sont combinées avec l'ADN viral de la vaccine, le procédé comprenant la combinaison des séquences d'ADN avec l'ADN viral de la vaccine.

43. Procédé selon la revendication 42, caractérisé en ce que le vaccin vivant contient un vecteur de la vaccine selon la revendication 29.

44. Procédé pour la fabrication d'un vaccin qui contient une composition selon la revendication 40, par combinaison d'un ou plusieurs des peptides ou polypeptides avec des adjuvants habituels.

45. Utilisation de l'un des vaccins selon les revendications 41 à 44 pour la production d'immunoglobulines spécifiques.

46. Agent diagnostique, caractérisé en ce qu'il contient une composition selon la revendication 40.

47. Procédé pour la fabrication d'une sonde d'ADN ou d'ARN qui hybride dans des conditions stringentes avec des séquences d'ADN ou d'ARN selon l'une des revendications 1 à 9 et 12 à 20, par technique de l'ARN/ADN recombinant ou par synthèse chimique.
